# EUROPEAN PATENT APPLICATION

(11) **EP 4 700 004 A1**
(43) Date of publication of application: **25.02.2026**
(21) Application number: 24792745.2
(22) Date of filing: 19.04.2024
(51) Int. Cl.: C07C 13/61, C07B 61/00, C07C 2/52, C07C 7/04, C07C 13/43, C07C 29/141, C07C 31/27, C07C 35/37, C07C 45/50, C07C 47/347

(54) **CYCLIC DIENE-CONTAINING COMPOSITION, PRODUCTION METHOD FOR ALDEHYDE, PRODUCTION METHOD FOR ALCOHOL, AND PRODUCTION METHOD FOR CYCLIC DIENE-CONTAINING COMPOSITION**

(30) Priority: 21.04.2023 JP 2023070230; 09.05.2023 JP 2023077353; 04.10.2023 JP 2023173116
(71) Applicant: Mitsubishi Chemical Corporation, Tokyo 100-8251 (JP)
(72) Inventor: SUNAMI, Kazuaki, Tokyo 100-8251 (JP); TASHIMA, Naoto, Tokyo 100-8251 (JP); HINOISHI, Hiroki, Tokyo 100-8251 (JP)
(74) Representative: Hoffmann Eitle
(86) International application number: PCT/JP2024/015499
(87) International publication number: WO 2024/219473

(57) **Abstract**

A cyclic diene-containing composition having a content of a cyclic diene of 99.5 GC area% or less and a content of a cyclic monoene of 3.0 GC area% or less. A method for producing an aldehyde, the method including subjecting a cyclic diene in a cyclic diene-containing composition to a hydroformylation reaction to produce a corresponding aldehyde, wherein the method includes controlling a content of a cyclic monoene contained in the cyclic diene-containing composition to a predetermined threshold (1) or less. A method for producing a cyclic diene-containing composition, the method including performing distillation and purification of a hydrocarbon decomposition product obtained by thermal decomposition of a hydrocarbon-containing composition to produce a cyclic diene-containing composition containing a cyclic diene, wherein the method includes controlling a content of a cyclic monoene contained in the cyclic diene-containing composition to a predetermined threshold (1) or less.

## Description

### Technical Field

The present invention relates to a cyclic diene-containing composition, a method for producing an aldehyde, a method for producing an alcohol, and a method for producing a cyclic diene-containing composition.

### Background Art

Aldehydes such as alicyclic aldehydes are useful as raw materials of additives for plasticizers, adhesives, microbicides, and the like. Furthermore, alcohols such as alicyclic alcohols obtained by subjecting alicyclic aldehydes to hydrogenation reaction are useful as raw material monomers for achieving higher heat resistance, flex resistance, or lower retardation of polyurethane-based resins, polyester-based resins, or polycarbonate-based resins. Specifically, alicyclic aldehydes such as tricyclodecane dicarbaldehyde and alicyclic alcohols such as tricyclodecane dimethanol and pentacyclopentadecane dimethanol have attracted attention from the viewpoint of achieving higher functionality in various applications and excellent industrial productivity.

In a known method for producing an alicyclic aldehyde such as tricyclodecane dicarbaldehyde, a C5 hydrocarbon fraction obtained by thermal decomposition of a hydrocarbon-containing composition such as naphtha, coal, or natural gas is heated for a dimerization reaction of a monocyclic diene such as cyclopentadiene in the C5 hydrocarbon fraction to form a corresponding polycyclic diene such as dicyclopentadiene, then the C5 hydrocarbon fraction after the dimerization reaction is purified to produce a cyclic diene-containing composition containing the polycyclic diene such as dicyclopentadiene at a high concentration, and the produced cyclic diene-containing composition is subjected to a hydroformylation reaction to convert it into an alicyclic aldehyde corresponding to the cyclic diene.

As a method for producing an alicyclic aldehyde by subjecting the cyclic diene-containing composition described above to a hydroformylation reaction, for example, Patent Literature 1 discloses, in Examples thereof, a technique for producing bisformyltricyclodecane by hydroformylation of highly purified dicyclopentadiene.

Patent Literature 2 discloses a technique for reducing conjugated diene, focusing on the conjugated diene contained in dicyclopentadiene as an impurity that inhibits a hydroformylation reaction.

Patent Literature 3 describes that dicyclopentadiene or tricyclopentadiene serving as a starting material preferably has high purity to produce tricyclodecane dicarbaldehyde or pentacyclopentadecane dicarbaldehyde, and discloses a technique for removing impurities such as butadiene, isoprene, cyclopentadiene, and 1,3-pentadiene.

However, the techniques disclosed in Patent Literatures 1 to 3 have problems, such as increased complication of production process, increased investment in plant and equipment and production cost, and increased utility cost, to separate and recover a highly pure cyclic diene by removing, as much as possible, impurities contained in a composition containing a polycyclic diene such as dicyclopentadiene (also referred to as "cyclic diene" in the present invention) (hereinafter the composition will also be referred to as "cyclic diene-containing composition"). Furthermore, even for reducing impurities contained in the cyclic diene-containing composition taking economic efficiency into consideration, impurities affecting the hydroformylation reaction are not fully understood.

Furthermore, a purified cyclic diene having high purity has a problem in that it is a solid at normal temperature and has poor industrial handleability. For example, a highly pure dicyclopentadiene has a melting point of 32.5°C and is a white crystalline solid at normal temperature; however, the dicyclopentadiene is preferably liquid in consideration of its handleability to industrially use it as a starting material for synthesis of various compounds.

### Citation List

### Patent Literature

Patent Literature 1: JP 2005-139181 A
Patent Literature 2: JP 11-80067 A
Patent Literature 3: JP 2001-11008 A

### Summary of Invention

### Technical Problem

An object of the present invention is to solve these problems.

That is, an object of the present invention is to provide a cyclic diene-containing composition that is used as a starting material for an alicyclic aldehyde and can produce the alicyclic aldehyde in a short reaction time and at a high yield.

Furthermore, another object of the present invention is to provide a cyclic diene-containing composition that is liquid at normal temperature and exhibits excellent industrial handleability.

Furthermore, another object of the present invention is to provide a method for producing a cyclic diene-containing composition containing a cyclic diene such as dicyclopentadiene, the cyclic diene being used as a starting material for an alicyclic aldehyde such as tricyclodecane dicarbaldehyde, the method being capable of producing an alicyclic aldehyde corresponding to the cyclic diene in a short reaction time and at a high yield.

Furthermore, another object of the present invention is to provide a method for producing an aldehyde, the method including producing an aldehyde corresponding to the cyclic diene using the cyclic diene-containing composition.

Alternatively, another object of the present invention is to provide a method for producing an aldehyde, the method using a cyclic diene-containing composition containing a cyclic diene such as dicyclopentadiene as a starting material and being capable of producing an alicyclic aldehyde corresponding to the cyclic diene, such as tricyclodecane dicarbaldehyde, in a short reaction time and at a high yield.

Alternatively, another object of the present invention is to provide a method for producing an aldehyde, the method including producing a cyclic diene-containing composition by the method for producing the cyclic diene-containing composition of the present invention, and producing a corresponding aldehyde from a cyclic diene contained in the cyclic diene-containing composition.

Furthermore, another object of the present invention is to provide a method for producing an alcohol, the method including producing an aldehyde by the method for producing the aldehyde described above, and producing a corresponding alcohol from the aldehyde.

### Solution to Problem

As a result of diligent research to solve the problems described above, the present inventors have found that the problems described above can be solved by controlling the content of a cyclic monoene contained in a cyclic diene-containing composition and the contents of a high-boiling-point byproduct and a cyclic diene contained in the composition.

That is, the gist of the present invention is as follows.
[1] A cyclic diene-containing composition having a content of a cyclic diene of 99.5 GC area% or less and a content of a cyclic monoene of 14.0 GC area% or less.
[2] The cyclic diene-containing composition according to [1], wherein a content of a high-boiling-point byproduct having a higher boiling point than the cyclic diene is 0.3 GC area% or more.
[3] The cyclic diene-containing composition according to [1] or [2], wherein the cyclic monoene contains a vinyl group.
[4] The cyclic diene-containing composition according to [3], wherein the cyclic monoene contains a vinyl group-containing cyclic monoene having 9 carbons.
[5] The cyclic diene-containing composition according to [4], wherein a content of the vinyl group-containing cyclic monoene having 9 carbons is 3.0 GC area% or less.
[6] The cyclic diene-containing composition according to any of [1] to [5], wherein the content of the cyclic diene is 60 GC area% or more.
[7] The cyclic diene-containing composition according to any of [1] to [6], wherein the content of the cyclic monoene is 0.001 GC area% or more.
[8] The cyclic diene-containing composition according to any of [1] to [7], wherein a content of a high-boiling-point byproduct having a higher boiling point than the cyclic diene is 25 GC area% or less.
[9] The cyclic diene-containing composition according to any of [2] to [8], wherein the content of the high-boiling-point byproduct is measured by measurement method 1 described below.

### Measurement method 1

The method includes measurement of a total content of a peak at a retention time from 15.0 minutes to 30.0 minutes by analysis under GC measurement conditions described below.

### GC measurement conditions

GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C

[10] The cyclic diene-containing composition according to any of [1] to [9], wherein the cyclic diene-containing composition has fluidity at 25°C.

[11] The cyclic diene-containing composition according to any of [1] to [10], wherein the cyclic diene is a polycyclic diene.

[12] The cyclic diene-containing composition according to [11], wherein the polycyclic diene is dicyclopentadiene.

[13] The cyclic diene-containing composition according to any of [1] to [12], wherein the cyclic monoene is a polycyclic monoene.

[14] The cyclic diene-containing composition according to [13], wherein the polycyclic monoene contains 5-vinyl-2-norbornene.

[15] The cyclic diene-containing composition according to any of [1] to [14], wherein the cyclic diene-containing composition is a composition produced by purification and separation of a hydrocarbon decomposition product obtained by thermal decomposition of a hydrocarbon-containing composition.

[16] The cyclic diene-containing composition according to [15], wherein the hydrocarbon-containing composition is naphtha.

[17] A method for producing an aldehyde, the method including subjecting the cyclic diene-containing composition described in any of [1] to [16] to a hydroformylation reaction to produce an aldehyde corresponding to the cyclic diene.

[18] A method for producing an alcohol, the method including producing an aldehyde by the method described in [17], and producing a corresponding alcohol from the aldehyde.

[19] A method for producing an aldehyde,
the method including subjecting a cyclic diene in a cyclic diene-containing composition to a hydroformylation reaction to produce a corresponding aldehyde, wherein
the method includes controlling a content of a cyclic monoene contained in the cyclic diene-containing composition to a predetermined threshold (1) or less.

[20] The method for producing an aldehyde according to [19], wherein the predetermined threshold (1) is 14.0 GC area%.

[21] The method for producing an aldehyde according to [19] or [20], the method including controlling a content of a high-boiling-point byproduct contained in the cyclic diene-containing composition and having a higher boiling point than the cyclic diene to a predetermined threshold (2) or more.

[22] The method for producing an aldehyde according to [21], wherein the predetermined threshold (2) is 0.3 GC area%.

[23] The method for producing an aldehyde according to any of [19] to [22], wherein a content of the cyclic diene contained in the cyclic diene-containing composition is 99.5 GC area% or less.

[24] The method for producing an aldehyde according to any of [19] to [23], wherein the cyclic monoene contains a vinyl group.

[25] The method for producing an aldehyde according to [24], wherein the cyclic monoene contains a vinyl group-containing cyclic monoene having 9 carbons.

[26] The method for producing an aldehyde according to [25], wherein a content of the vinyl group-containing cyclic monoene having 9 carbons in the cyclic diene-containing composition is 3.0 GC area% or less.

[27] The method for producing an aldehyde according to any of [19] to [26], wherein a content of the cyclic diene contained in the cyclic diene-containing composition is 60 GC area% or more.

[28] The method for producing an aldehyde according to any of [19] to [27], wherein a content of the cyclic monoene contained in the cyclic diene-containing composition is 0.001 GC area% or more.

[29] The method for producing an aldehyde according any of [19] to [28], wherein a content of a high-boiling-point byproduct contained in the cyclic diene-containing composition and having a higher boiling point than the cyclic diene is 25 GC area% or less.

[30] The method for producing an aldehyde according to any of [21] to [29], wherein a content of the high-boiling-point byproduct is measured by measurement method 1 described below.

### Measurement method 1

The method includes measurement of a total content of a peak at a retention time from 15.0 minutes to 30.0 minutes by analysis under GC measurement conditions described below.

### GC measurement conditions

GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C

[31] The method for producing an aldehyde according to any of [19] to [30], wherein the cyclic diene is a polycyclic diene.

[32] The method for producing an aldehyde according to [31], wherein the polycyclic diene is dicyclopentadiene.

[33] The method for producing an aldehyde according to any of [19] to [32], wherein the cyclic monoene is a polycyclic monoene.

[34] The method for producing an aldehyde according to [32], wherein the polycyclic monoene contains 5-vinyl-2-norbornene.

[35] The method for producing an aldehyde according to any of [19] to [34], wherein the cyclic diene-containing composition is a composition produced by distillation and purification of a hydrocarbon decomposition product obtained by thermal decomposition of a hydrocarbon-containing composition.

[36] The method for producing an aldehyde according to [35], wherein the hydrocarbon-containing composition is naphtha.

[37] The method for producing an aldehyde according to [35] or [36], wherein the method includes controlling a condition of the distillation and purification so that the content of the cyclic monoene contained in the cyclic diene-containing composition is adjusted to the predetermined threshold (1) or less.

[38] The method for producing an aldehyde according to any of [35] to [37], wherein the method includes controlling a condition of the distillation and purification so that a content of the high-boiling-point byproduct contained in the cyclic diene-containing composition is adjusted to a predetermined threshold (2) or more.

[39] The method for producing an aldehyde according to any of [35] to [38], wherein, in the distillation and purification, a first distillation column and a second distillation column are included, the number of theoretical plates of the first distillation column is 10 or more and 20 or less, and the number of theoretical plates of the second distillation column is 10 or more and 20 or less.

[40] The method for producing an aldehyde according to [39], wherein a reflux ratio of the first distillation column is 20 or more and 30 or less, and a reflux ratio of the second distillation column is 1.0 or more and 1.5 or less.

[41] A method for producing an alcohol, the method including producing an aldehyde by the method described in any of [19] to [38], and producing a corresponding alcohol from the aldehyde.

[42] A method for producing an alcohol, the method including producing an aldehyde by the method described in [39] or [40], and producing a corresponding alcohol from the aldehyde.

[43] A method for producing a cyclic diene-containing composition, the method including performing distillation and purification of a hydrocarbon decomposition product obtained by thermal decomposition of a hydrocarbon-containing composition to produce a cyclic diene-containing composition containing a cyclic diene, wherein the method includes controlling a content of a cyclic monoene contained in the cyclic diene-containing composition to a predetermined threshold (1) or less.

[44] The method for producing a cyclic diene-containing composition according to [43], wherein the method includes controlling a content of a high-boiling-point byproduct contained in the cyclic diene-containing composition and having a higher boiling point than the cyclic diene to a predetermined threshold (2) or more.

[45] The method for producing a cyclic diene-containing composition according to [43] or [44], wherein the predetermined threshold (1) is 14.0 GC area%.

[46] The method for producing a cyclic diene-containing composition according to [44] or [45], wherein the predetermined threshold (2) is 0.3 GC area%.

[47] The method for producing a cyclic diene-containing composition according to any of [43] to [46], wherein the method includes controlling a condition of the distillation and purification so that the content of the cyclic monoene contained in the cyclic diene-containing composition is adjusted to the predetermined threshold (1) or less.

[48] The method for a cyclic diene-containing composition according to any of [44] to [47], wherein the method includes controlling a condition of the distillation and purification so that the content of the high-boiling-point byproduct contained in the cyclic diene-containing composition is adjusted to the predetermined threshold (2) or more.

[49] The method for producing a cyclic diene-containing composition according to any of [43] to [48], wherein, in the distillation and purification, a first distillation column and a second distillation column are included, the number of theoretical plates of the first distillation column is 10 or more and 20 or less, and the number of theoretical plates of the second distillation column is 10 or more and 20 or less.

[50] The method for producing a cyclic diene-containing composition according to [49], wherein a reflux ratio of the first distillation column is 20 or more and 30 or less, and a reflux ratio of the second distillation column is 1.0 or more and 1.5 or less.

[51] The method for producing a cyclic diene-containing composition according to any of [43] to [50], wherein a content of the cyclic diene contained in the cyclic diene-containing composition is 99.5 GC area% or less.

[52] The method for producing a cyclic diene-containing composition according to any of [43] to [51], wherein the cyclic monoene contains a vinyl group.

[53] The method for producing a cyclic diene-containing composition according to [52], wherein the cyclic monoene contains a vinyl group-containing cyclic monoene having 9 carbons.

[54] The method for producing a cyclic diene-containing composition according to [53], wherein a content of the vinyl group-containing cyclic monoene having 9 carbons in the cyclic diene-containing composition is 3.0 GC area% or less.

[55] The method for producing a cyclic diene-containing composition according to any of [43] to [54], wherein a content of the cyclic diene contained in the cyclic diene-containing composition is 60.0 GC area% or more.

[56] The method for producing a cyclic diene-containing composition according to any of [43] to [55], wherein a content of the cyclic monoene contained in the cyclic diene-containing composition is 0.001 GC area% or more.

[57] The method for producing a cyclic diene-containing composition according to any of [43] to [56], wherein a content of a high-boiling-point byproduct contained in the cyclic diene-containing composition and having a higher boiling point than the cyclic diene is 25 GC area% or less.

[58] The method for producing a cyclic diene-containing composition according to any of [44] to [57], wherein a content of the high-boiling-point byproduct is measured by measurement method 1 described below.

### Measurement method 1

The method includes measurement of a total content of a peak at a retention time from 15.0 minutes to 30.0 minutes by analysis under GC measurement conditions described below.

### GC measurement conditions

GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C

[59] The method for producing a cyclic diene-containing composition according to any of [43] to [58], wherein the cyclic diene is a polycyclic diene.

[60] The method for producing a cyclic diene-containing composition according to [59], wherein the polycyclic diene is dicyclopentadiene.

[61] The method for producing a cyclic diene-containing composition according to any of [43] to [60], wherein the cyclic monoene is a polycyclic monoene.

[62] The method for producing a cyclic diene-containing composition according to [61], wherein the polycyclic monoene contains 5-vinyl-2-norbornene.

[63] The method for producing a cyclic diene-containing composition according to any of [43] to [62], wherein the hydrocarbon-containing composition is naphtha.

[64] A method for producing an aldehyde, the method including producing a cyclic diene-containing composition by the method described in any of [43] to [48] and [51] to [63], and producing a corresponding aldehyde from a cyclic diene contained in the cyclic diene-containing composition.

[65] A method for producing an alcohol, the method including producing an aldehyde by the method described in [64], and producing a corresponding alcohol from the aldehyde.

[66] A method for producing an aldehyde, the method including producing a cyclic diene-containing composition by the method described in [49] or [50], and producing a corresponding aldehyde from a cyclic diene contained in the cyclic diene-containing composition.

[67] A method for producing an alcohol, the method including producing an aldehyde by the method described in [66], and producing a corresponding alcohol from the aldehyde.

### Advantageous Effects of Invention

According to the present invention, there is provided a cyclic diene-containing composition that is used as a starting material for an alicyclic aldehyde and can efficiently produce the alicyclic aldehyde. By using this cyclic diene-containing composition, corresponding aldehyde and alcohol can be efficiently produced.

Furthermore, the cyclic diene-containing composition of the present invention can be liquid at normal temperature, and thus a cyclic diene-containing composition having excellent industrial handleability can be provided.

Furthermore, according to the present invention, there can be provided a method for producing an aldehyde, the method using a cyclic diene-containing composition containing a cyclic diene such as dicyclopentadiene as a starting material and being capable of producing an alicyclic aldehyde corresponding to the cyclic diene, such as tricyclodecane dicarbaldehyde, in a short reaction time and at a high yield. In addition, there can be provided a method for producing an alcohol, the method producing a corresponding alcohol from the aldehyde.

Furthermore, according to the method for producing an aldehyde of the present invention, the cyclic diene-containing composition used as a starting material can be liquid at normal temperature, and thus a method for producing an aldehyde having excellent operability can be provided.

Furthermore, according to the present invention, there can be provided a method for producing a cyclic diene-containing composition containing a cyclic diene such as dicyclopentadiene used as a starting material for an alicyclic aldehyde such as tricyclodecane dicarbaldehyde, the method being capable of producing an alicyclic aldehyde corresponding to the cyclic diene in a short reaction time and at a high yield. In addition, there can be provided a method for producing an aldehyde, the method producing a corresponding aldehyde from the cyclic diene contained in the cyclic diene-containing composition, and a method for producing an alcohol, the method producing a corresponding alcohol from the aldehyde.

Furthermore, according to the method for producing a cyclic diene-containing composition of the present invention, the resulting cyclic diene-containing composition can be liquid at normal temperature, and thus a cyclic diene-containing composition having excellent industrial handleability can be provided.

### Brief Description of Drawings

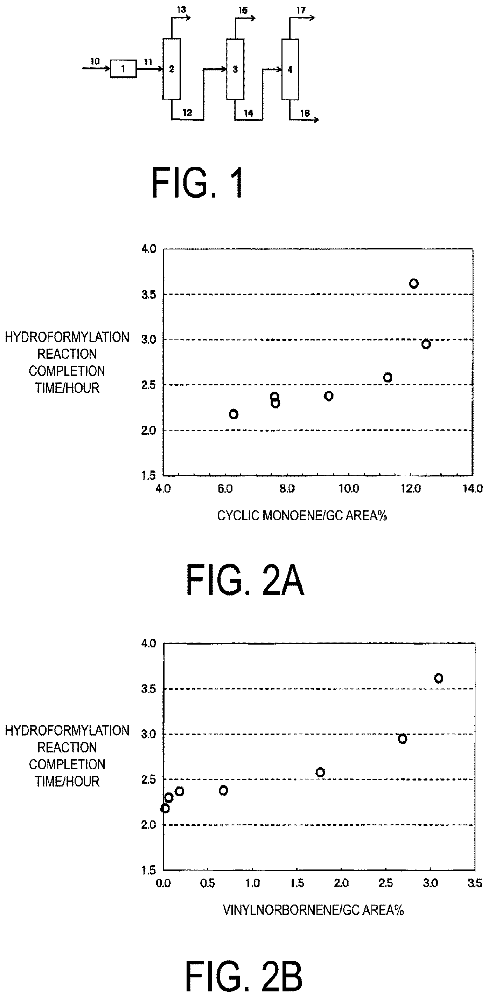

FIG. 1 is a schematic system diagram illustrating an embodiment of a production process in which a cyclic diene-containing composition of the present invention is separated and recovered from a C5 hydrocarbon fraction according to the present invention.
FIG. 2A is a graph illustrating the relationship between the content (GC area%) of cyclic monoenes (total of vinylnorbornene and isopropenylnorbornene) contained in each of cyclic diene-containing compositions (fractions 1 to 7) and hydroformylation reaction completion time when fractions 1 to 7 used in Experimental Examples 1 to 7 were subjected to a hydroformylation reaction.
FIG. 2B is a graph illustrating the relationship between the content (GC area%) of vinylnorbornene contained in each of cyclic diene-containing compositions (fractions 1 to 7) and hydroformylation reaction completion time when fractions 1 to 7 used in Experimental Examples 1 to 7 were subjected to a hydroformylation reaction.

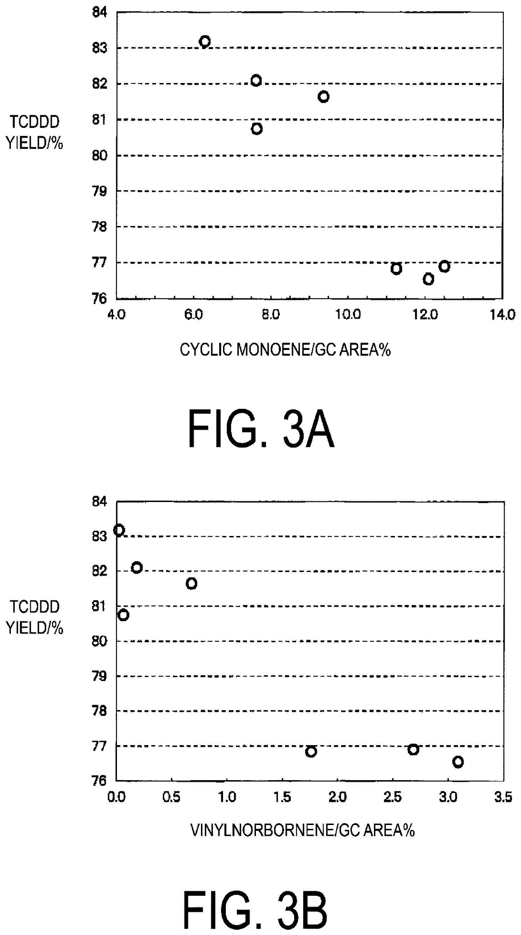

FIG. 3A is a graph illustrating the relationship between the content (GC area%) of cyclic monoenes (total of vinylnorbornene and isopropenylnorbornene) contained in each of cyclic diene-containing compositions (fractions 1 to 7) and the TCDDD yield in hydroformylation reaction when fractions 1 to 7 used in Experimental Examples 1 to 7 were subjected to a hydroformylation reaction.
FIG. 3B is a graph illustrating the relationship between the content (GC area%) of vinylnorbornene contained in each of cyclic diene-containing compositions (fractions 1 to 7) and the TCDDD yield in hydroformylation reaction when fractions 1 to 7 used in Experimental Examples 1 to 7 were subjected to a hydroformylation reaction.

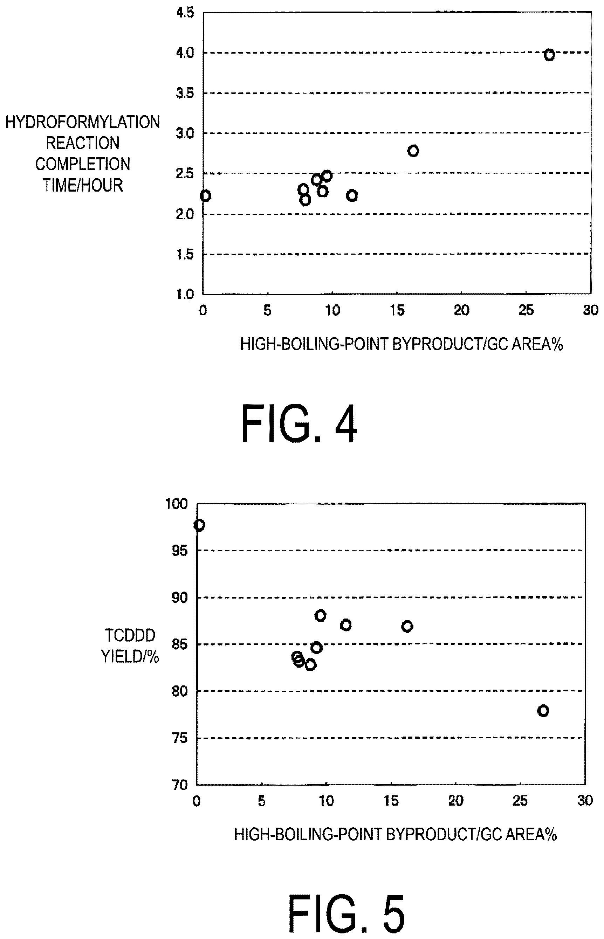

FIG. 4 is a graph illustrating the relationship between the content (GC area%) of a high-boiling-point byproduct contained in each of cyclic diene-containing compositions (fractions 7 to 14, and commercially available DCPD) and hydroformylation reaction completion time when fractions 7 to 14 used in Experimental Examples 7 to 14 and a commercially available DCPD used in Example 15 were subjected to a hydroformylation reaction.
FIG. 5 is a graph illustrating the relationship between the content (GC area%) of a high-boiling-point byproduct contained in each of cyclic diene-containing compositions (fractions 7 to 14, and commercially available DCPD) and TCDDD yield when fractions 7 to 14 used in Experimental Examples 7 to 14 and a commercially available DCPD used in Example 15 were subjected to a hydroformylation reaction.

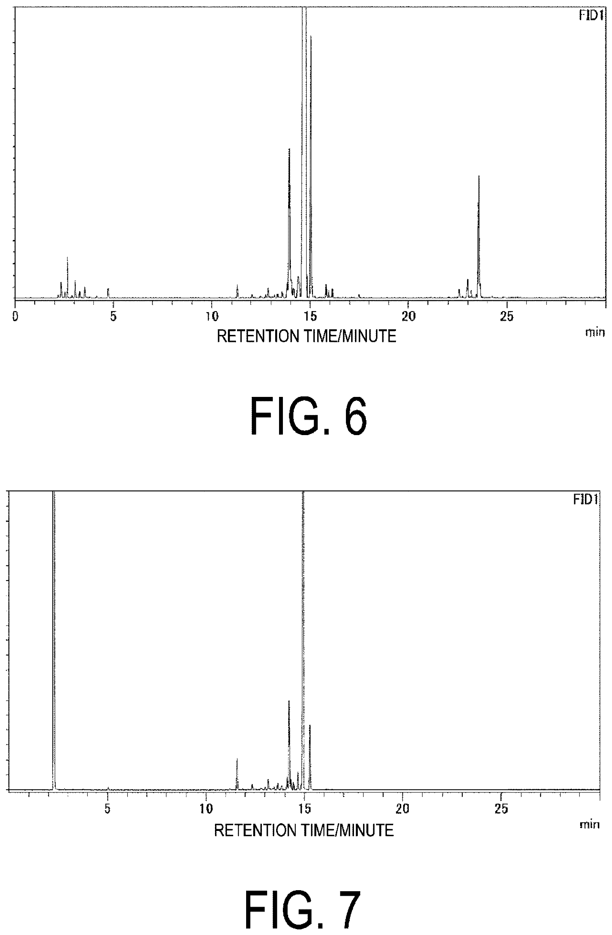

FIG. 6 is a gas chromatogram of a C5 fraction heavy composition obtained in Reference Experimental Example 1.
FIG. 7 is a gas chromatogram of a fraction 2 obtained in Reference Experimental Example 2.

### Description of Embodiments

Hereinafter, the present invention will be described in detail. The present invention is not limited to the following description and can be carried out with optional modifications within a scope that does not depart from the gist of the present invention.

Unless otherwise noted, a numerical range represented by the term "to" in the present specification means a range including the numerical values written before and after "to" as the lower limit value and the upper limit value, respectively, and "A to B" means A or more and B or less.

In the present specification, unless otherwise noted, "containing A or B" means "containing A", "containing B", and "containing A and B".

In the present specification, the term "GC area%" refers to a compositional proportion of each component measured by using a gas chromatograph (GC) and a gas chromatography total area method and calculated as an area content (unit: GC area%) of each peak component when the total area of GC peaks of all products in a gas chromatogram is taken as 100%. Details of the GC measurement conditions will be described in Experimental Examples below.

In the present specification, the term "mass%" refers to the content of a predetermined component contained in 100 mass% total. The term "wt.%" refers to the content of a predetermined component contained in 100 wt.% total.

The terms "mass%" and "wt.%" are synonymous with each other.

The term "optional" or "optionally" means that the subsequently described situation may occur or may not occur. Thus, the description includes a case where the situation occurs and a case where the situation does not occur.

The term "normal temperature" used in the present specification refers to a temperature of 25°C.

The term "approximately" used in the present specification can mean ± 20% of the indicated value. For example, approximately 75°C includes a range of 60°C to 90°C.

All steps described in the present specification can be performed in any suitable order unless otherwise noted in the present specification or unless clearly contradict the context.

In the present specification, "hydrocarbon" means naphtha, coal, and natural gas. More specifically, "hydrocarbon" means hydrocarbons including, for example, fossil fuels and refined petroleum products, such as naphtha, heavy aromatic naphtha, crude oil, gasoline, kerosene, diesel oil, light oil, heavy oil, extra heavy oil, heavy oil A, heavy oil B, heavy oil C, jet fuel oil, tar, gas-to-liquid oil (GTL), coal-to-liquid oil (CTL), coal, coke, natural gas, liquefied natural gas (LNG), liquefied petroleum gas (LPG), sour gas, oilfield gas, and oilfield concentrates.

Next will described a cyclic diene-containing composition, i.e., a first embodiment of the present invention, a method for producing an aldehyde, i.e., a second embodiment of the present invention, and a method for producing a cyclic diene-containing composition, i.e., a third embodiment of the present invention (hereinafter, the first to third embodiments of the invention may be collectively referred to as "the present invention").

### Cyclic Diene-Containing Composition

The cyclic diene-containing composition according to the first embodiment of the present invention will be described below.

### Embodiment 1-1

The cyclic diene-containing composition according to the embodiment 1-1 of the present invention is a composition containing a cyclic diene described below and having a content of the cyclic diene of 99.5 GC area% or less and a content of a cyclic monoene described below of 14.0 GC area% or less per 100% of the total GC area of the cyclic diene-containing composition. The details of the content of the cyclic monoene in the cyclic diene-containing composition will be described below.

Furthermore, the cyclic diene-containing composition of the present invention may contain a high-boiling-point byproduct described below. The content of the high-boiling-point byproduct in the cyclic diene-containing composition is preferably 0.3 GC area% or more per 100% of the total GC area of the cyclic diene-containing composition. The details of the content of the high-boiling-point byproduct in the cyclic diene-containing composition will be described below.

### Embodiment 1-2

The cyclic diene-containing composition according to the embodiment 1-2 of the present invention is a cyclic diene-containing composition produced by the method for producing a cyclic diene-containing composition, which is the third embodiment of the present invention described below.

The cyclic diene-containing compositions according to the embodiments 1-1 and 1-2 of the present invention will be collectively referred to simply as the "cyclic diene-containing composition of the present invention".

In the present specification, the cyclic diene-containing composition of the present invention, the cyclic diene-containing composition according to the method for producing an aldehyde, which is the second embodiment of the present invention, and the cyclic diene-containing composition according to the method for producing a cyclic diene-containing composition, which is the third embodiment of the present invention will be collectively referred to as "the cyclic diene-containing composition of the present invention". That is, the "cyclic diene-containing compositions" according to the first to third embodiments of the present invention can be treated as being synonymous to one another.

### Cyclic Diene

The cyclic diene (also referred to as "cyclic diene") of the present invention is a component constituting the cyclic diene-containing composition of the present invention.

The cyclic diene of the present invention is not particularly limited as long as the cyclic diene is a compound having a ring structure in the molecule and having two non-conjugated carbon-carbon double bonds in the ring structure. The cyclic diene is preferably a compound that can be used as a starting material for producing an alicyclic alcohol.

An embodiment of the cyclic diene is a polycyclic diene having two or more ring structures in the molecule wherein the two ring structures each have a non-conjugated carbon-carbon double bond.

Alternatively, another embodiment of the cyclic diene is a compound obtained by dimerization of a monocyclic diene having one ring structure.

Specific examples of the cyclic diene include dicyclopentadiene, 1,4-cyclohexadiene, methylcyclopentadiene, methyldicyclopentadiene, and methyltetrahydroindene.

Among these cyclic dienes, dicyclopentadiene is preferred from the viewpoints of industrial availability, excellent hydroformylation reactivity, and excellent production efficiency of an alicyclic alcohol.

One type of these cyclic dienes can be used alone, or two or more types of these cyclic dienes can be used in combination.

In the present specification, the cyclic diene according to the cyclic diene-containing composition of the present invention, the cyclic diene according to the method for producing an aldehyde, which is the second embodiment of the present invention, and the cyclic diene according to the method for producing a cyclic diene-containing composition, which is the third embodiment of the present invention will be collectively referred to as "the cyclic diene of the present invention".

That is, the "cyclic dienes" according to the first to third embodiments of the present invention can be treated as being synonymous to one another.

The upper limit of the content (unit: GC area%) of the cyclic diene in the cyclic diene-containing composition of the present invention is preferably 99.5% or less, more preferably 97.5% or less, even more preferably 95.0% or less, particularly preferably 90.0% or less, and most preferably 85.0% or less, per 100% of the total GC area of the cyclic diene-containing composition, from the viewpoints of having fluidity at normal temperature, i.e., being liquid at normal temperature, and having excellent industrial handleability.

Meanwhile, although the lower limit of the content (unit: GC area%) of the cyclic diene is not particularly limited, from the viewpoint of producing an alicyclic aldehyde by using the cyclic diene-containing composition as a starting material in a short reaction time and at a high yield, the lower limit is preferably 60.0% or more, more preferably 63.0% or more, even more preferably 66.0% or more, particularly preferably 70.0% or more, and most preferably 75.0% or more, per 100% of the total GC area of the cyclic diene-containing composition.

The aforementioned upper and lower limits can be freely combined. For example, the content (unit: GC area%) of the cyclic diene in the cyclic diene-containing composition is preferably 60.0% or more and 99.5% or less, more preferably 63.0% or more and 97.5% or less, even more preferably 66.0% or more and 95.0% or less, particularly preferably 70.0% or more and 90.0% or less, and most preferably 75.0% or more and 85.0% or less, per 100% of the total GC area of the cyclic diene-containing composition.

The measurement method of the content of the cyclic diene in the cyclic diene-containing composition will be described in detail below.

Examples of the method of controlling the content of the cyclic diene in the cyclic diene-containing composition include a method of controlling the amount by an extraction method, purification conditions, or distillation conditions, and a method of combining these. Preferred is a method of controlling the distillation and purification conditions.

### Cyclic Monoene

By setting the content of the cyclic monoene (also abbreviated as "cyclic monoene") of the cyclic diene-containing composition of the present invention to the predetermined threshold (1) or less, when the cyclic diene-containing composition is used as a starting material for an alicyclic aldehyde, the alicyclic aldehyde can be produced in a short reaction time and at a high yield.

In the present specification, the cyclic monoene according to the cyclic diene-containing composition of the present invention, the cyclic monoene according to the method for producing an aldehyde, which is the second embodiment of the present invention, and the cyclic monoene according to the method for producing a cyclic diene-containing composition, which is the third embodiment of the present invention are collectively referred to as "the cyclic monoene of the present invention".

That is, the "cyclic monoenes" according to the first to third embodiments of the present invention can be treated as being synonymous to one another.

The cyclic monoene of the present invention is a compound having one or more ring structures in the molecule and having one carbon-carbon double bond contained in the ring structures, and more specifically a compound having one or more ring structures in the molecule, having one carbon-carbon double bond contained in the ring structures, and having one or more vinyl groups that are not contained in the ring structures.

An embodiment of the cyclic monoene is a polycyclic monoene having two or more ring structures in the molecule, having one carbon-carbon double bond contained in the ring structures, and having one vinyl group that is not contained in the ring structures.

Alternatively, another embodiment of the cyclic monoene of is a chiral compound of an alkenylnorbornene including one enantiomer represented by General Formula (1) described below.

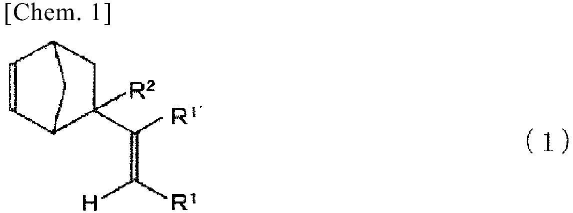

(In Formula (1), R¹ and R^{1'} each independently represent a hydrogen atom or an alkyl group having from 1 to 10 carbons, being optionally branched, and optionally having a substituent. R² represents a hydrogen atom or a methyl group.)

Examples of the cyclic monoene of the present invention include cyclic monoene having the vinyl group described above and having 9 carbons.

Specific examples of the cyclic monoene include 5-vinyl-2-norbornene (referred to as "vinylnorbornene" in the present specification), isopropenyl-2-norbornene (referred to as "isopropenylnorbornene" in the present specification), 2-(1-propenyl)-5-norbornene, 5-(1-methylenepropyl)norbornene, 5-(2-methyl-1-methylenepropyl)norbornene, and 5-(1-phenylethenyl)norbornene.

In a case where the cyclic diene described above is dicyclopentadiene, examples of the cyclic monoene include 5-vinyl-2-norbornene and isopropenyl-2-norbornene.

In a case where the cyclic monoene has optical activity, the cyclic monoene of the present invention may be a single substance of one of enantiomers or may be a racemic mixture of the enantiomers.

The upper limit of the content (unit: GC area%) of the cyclic monoene in the cyclic diene-containing composition of the present invention is preferably 14.0% or less, more preferably 12.0% or less, even more preferably 10.0% or less, particularly preferably 8.0% or less, and most preferably 7.0% or less, per 100% of the total GC area of the cyclic diene-containing composition, from the viewpoint of producing an alicyclic aldehyde by using the cyclic diene-containing composition as a starting material in a short reaction time and at a high yield.

Thus, from the viewpoints described above, the predetermined threshold (1) is preferably 14.0% or less, more preferably 12.0% or less, even more preferably 10.0% or less, particularly preferably 8.0% or less, and most preferably 7.0% or less, per 100% of the total GC area of the cyclic diene-containing composition.

Thus, in the method for producing an aldehyde, which is the second embodiment of the present invention, the threshold (1) of the content of the cyclic monoene in the cyclic diene-containing composition is preferably adjusted to be equal to or lower than the upper limit described above to control the content of the cyclic monoene in the cyclic diene-containing composition.

In the method for producing a cyclic diene-containing composition, which is the third embodiment of the present invention, the threshold (1) of the content of the cyclic monoene in the cyclic diene-containing composition is preferably adjusted to be equal to or lower than the upper limit described above to control the content of the cyclic monoene in the cyclic diene-containing composition.

Meanwhile, the lower limit of the content (unit: GC area%) of the cyclic monoene in the cyclic diene-containing composition is not particularly limited, and substantially no cyclic monoene may be contained (0.0%); however, from the viewpoint of economic efficiency such as production cost required for purification and separation of the cyclic diene-containing composition, the lower limit is preferably 0.001% or more, more preferably 0.003% or more, even more preferably 0.01% or more, particularly preferably 0.03% or more, and most preferably 0.1% or more, per 100% of the total GC area of the cyclic diene-containing composition.

The aforementioned upper and lower limits can be freely combined. For example, the cyclic monoene may be substantially not contained (0.0%), or the content (unit: GC area%) of the cyclic monoene in the cyclic diene-containing composition is preferably 0.001% or more and 14.0% or less, more preferably 0.003% or more and 12.0% or less, even more preferably 0.01% or more and 10.0% or less, particularly preferably 0.03% or more and 8.0% or less, and most preferably 0.1% or more and 7.0% or less, per 100% of the total GC area of the cyclic diene-containing composition.

Furthermore, the upper limit of the content (unit: GC area%) of the vinyl group-containing cyclic monoene having 9 carbons, such as vinylnorbornene, in the cyclic diene-containing composition of the present invention is preferably 3.0% or less, more preferably 2.5% or less, even more preferably 2.0% or less, particularly preferably 1.5% or less, and most preferably 1.0% or less, per 100% of the total GC area of the cyclic diene-containing composition, from the viewpoint of producing an alicyclic aldehyde by using the cyclic diene-containing composition as a starting material in a short reaction time and at a high yield.

Thus, in a case of the vinyl group-containing cyclic monoene having 9 carbons, from the viewpoints described above, the predetermined threshold (1) is preferably 3.0%, more preferably 2.5%, even more preferably 2.0%, particularly preferably 1.5%, and most preferably 1.0%, per 100% of the total GC area of the cyclic diene-containing composition.

Thus, in the method for producing an aldehyde, which is the second embodiment of the present invention, the threshold (1) of the content of the vinyl group-containing cyclic monoene having 9 carbons, such as vinylnorbornene, is preferably adjusted to be equal to or lower than the upper limit described above to control the content of the cyclic monoene in the cyclic diene-containing composition.

In the method for producing a cyclic diene-containing composition, which is the third embodiment of the present invention, the threshold (1) of the content of the vinyl group-containing cyclic monoene having 9 carbons, such as vinylnorbornene, is preferably adjusted to be equal to or lower than the upper limit described above to control the content of the cyclic monoene in the cyclic diene-containing composition.

Meanwhile, the lower limit of the content (unit: GC area%) of the vinyl group-containing cyclic monoene having 9 carbons, such as vinylnorbornene, in the cyclic diene-containing composition is not particularly limited, and substantially no cyclic monoene may be contained (0.0%); however, from the viewpoint of economic efficiency such as production cost required for purification and separation of the cyclic diene-containing composition, the lower limit is preferably 0.001% or more, more preferably 0.003% or more, even more preferably 0.01% or more, particularly preferably 0.03% or more, and most preferably 0.1% or more, per 100% of the total GC area of the cyclic diene-containing composition.

The aforementioned upper and lower limits can be freely combined. For example, the vinyl group-containing cyclic monoene having 9 carbons, such as vinylnorbornene, may be substantially not contained (0.0%), or the content (unit: GC area%) of the vinyl group-containing cyclic monoene having 9 carbons, such as vinylnorbornene, is preferably 0.001% or more and 3.0% or less, more preferably 0.003% or more and 2.5% or less, even more preferably 0.01% or more and 2.0% or less, particularly preferably 0.03% or more and 1.5% or less, and most preferably 0.1% or more and 1.0% or less, per 100% of the total GC area of the cyclic diene-containing composition.

The measurement method of the content of the cyclic monoene in the cyclic diene-containing composition will be described in detail below.

Examples of the method of controlling the content of the cyclic monoene in the cyclic diene-containing composition include a method of controlling the amount by an extraction method, purification conditions, or distillation conditions, and a method of combining these. Preferred is a method of controlling the distillation and purification conditions.

### High-Boiling-Point Byproduct

The high-boiling-point byproduct in the present invention refers to a high-boiling-point byproduct that is formed as a byproduct at the time of production of the cyclic diene-containing composition of the present invention and that has a higher boiling point than the cyclic diene in the present invention (hereinafter simply referred to as "high-boiling-point byproduct").

Examples of the high-boiling-point byproduct include indane, a trimer of indene or cyclopentadiene, and a low polymer of a combination of these components. This high-boiling-point byproduct corresponds to, for example, a high-boiling-point component that is removed in a step (IV) described below as a specific embodiment of the method for producing a cyclic diene-containing composition of the present invention.

In the present specification, the high-boiling-point byproduct according to the cyclic diene-containing composition of the present invention, the high-boiling-point byproduct according to the method for producing an aldehyde, which is the second embodiment of the present invention, and the high-boiling-point byproduct according to the method for producing a cyclic diene-containing composition, which is the third embodiment of the present invention are collectively referred to as "the high-boiling-point byproduct of the present invention".

That is, the "high-boiling-point byproducts" of the first to third embodiments of the present invention can be treated as being synonymous to one another.

By controlling the content of the high-boiling-point byproduct, when the cyclic diene-containing composition of the present invention is used as a starting material for an alicyclic aldehyde, the cyclic diene-containing composition can achieve superior production efficiency of an alicyclic aldehyde, superior handleability of the cyclic diene-containing composition, and superior operability at the time of production of an aldehyde.

The upper limit of the content (unit: GC area%) of the high-boiling-point byproduct in the cyclic diene-containing composition of the present invention is preferably 25.0% or less, more preferably 20.0% or less, even more preferably 15.0% or less, particularly preferably 10.0% or less, and most preferably 8.0% or less, per 100% of the total GC area of the cyclic diene-containing composition, from the viewpoint of producing an alicyclic aldehyde by using the cyclic diene-containing composition as a starting material in a short reaction time and at a high yield.

Meanwhile, the lower limit of the content (unit: GC area%) of the high-boiling-point byproduct is not particularly limited, and substantially no high-boiling-point byproduct may be contained (0.0%); however, from the viewpoints of being liquid at normal temperature and achieving superior industrial handleability and from the viewpoint of economic efficiency such as production cost required for purification and separation of the cyclic diene-containing composition, the lower limit is preferably 0.3% or more, more preferably 0.6% or more, even more preferably 1.0% or more, particularly preferably 3.0% or more, and most preferably 5.0% or more, per 100% of the total GC area of the cyclic diene-containing composition.

Thus, in the method for producing an aldehyde, which is the second embodiment of the present invention described below, the threshold (2) of the content of the high-boiling-point byproduct in the cyclic diene-containing composition is preferably adjusted to be equal to or higher than the lower limit described above to control the content of the high-boiling-point byproduct in the cyclic diene-containing composition.

In the method for producing a cyclic diene-containing composition, which is the third embodiment of the present invention described below, the threshold (2) of the content of the high-boiling-point byproduct in the cyclic diene-containing composition is preferably adjusted to be equal to or higher than the lower limit described above to control the content of the high-boiling-point byproduct in the cyclic diene-containing composition.

The aforementioned upper and lower limits can be freely combined. For example, the high-boiling-point byproduct may be substantially not contained (0.0%), or the content (unit: GC area%) of the high-boiling-point byproduct in the cyclic diene-containing composition is preferably 0.3% or more and 25.0% or less, more preferably 0.6% or more and 20.0% or less, even more preferably 1.0% or more and 15.0% or less, particularly preferably 3.0% or more and 10.0% or less, and most preferably 5.0% or more and 8.0% or less, per 100% of the total GC area of the cyclic diene-containing composition.

The content of the high-boiling-point byproduct in the cyclic diene-containing composition of the present invention can be measured by the following measurement method 1. The details of the measurement method will be described below.

### Measurement Method 1

The total content of a peak at a retention time from 15.0 minutes to 30.0 minutes is measured by analysis under GC measurement conditions described below.

### GC Measurement Conditions

GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

Examples of the method of controlling the content of the high-boiling-point byproduct in the cyclic diene-containing composition include a method of controlling the amount by an extraction method, purification conditions, or distillation conditions, and a method of combining these. Preferred is a method of controlling the distillation and purification conditions.

### Fluidity

The cyclic diene-containing composition of the present invention preferably has fluidity at normal temperature (25°C).

"Having fluidity" means that, when a substance is placed in a container and the container is tilted, its shape changes due to flowing, and preferably means being "liquid".

That is, the cyclic diene-containing composition of the present invention is preferably liquid at normal temperature.

The cyclic diene-containing composition of the present invention can have fluidity at normal temperature by setting the content of the cyclic diene in the composition to be equal to or lower than the upper limit described above.

### Method for Producing Cyclic Diene-Containing Composition

The method for producing the cyclic diene-containing composition of the present invention is not particularly limited, and examples thereof include the method for producing a cyclic diene-containing composition, which is the third embodiment of the present invention.

The method for producing a cyclic diene-containing composition, which is the third embodiment of the present invention is a method for producing a cyclic diene-containing composition containing the cyclic diene of the embodiment 1-2 described above, the method including performing distillation and purification of a hydrocarbon decomposition product described below obtained by thermal decomposition of a hydrocarbon-containing composition described below, wherein the method includes controlling the content of a cyclic monoene contained in the cyclic diene-containing composition to a predetermined threshold (1) or less.

In the method for producing a cyclic diene-containing composition of the present invention, the method of obtaining the hydrocarbon decomposition product by thermal decomposition of the hydrocarbon-containing composition is not particularly limited. A specific embodiment is a method of obtaining a hydrocarbon decomposition product by thermal decomposition of a hydrocarbon-containing composition, such as naphtha, in treatment equipment of a hydrocarbon decomposition product, such as ethylene production equipment described below.

In the method for producing a cyclic diene-containing composition of the present invention, the method of obtaining the cyclic diene-containing composition containing the cyclic diene by distillation and purification of the hydrocarbon decomposition product is not particularly limited. A specific embodiment is a method of obtaining the cyclic diene-containing composition by performing separation and purification of the hydrocarbon decomposition product, obtaining a C5 hydrocarbon fraction described below, and performing distillation and purification of the obtained C5 hydrocarbon fraction.

By controlling the content of the cyclic monoene contained in the cyclic diene-containing composition to the predetermined threshold (1) or less, an alicyclic aldehyde corresponding to the cyclic diene can be produced in a short reaction time and at a high yield.

From the viewpoints described above, the predetermined threshold (1) is preferably 14.0% or less, more preferably 12.0% or less, even more preferably 10.0% or less, particularly preferably 8.0% or less, and most preferably 7.0% or less, per 100% of the total GC area of the cyclic diene-containing composition.

In a case where the cyclic monoene is a vinyl group-containing cyclic monoene having 9 carbons, such as vinylnorbornene, from the viewpoints described above, the predetermined threshold (1) is preferably 3.0 GC area%, more preferably 2.5 GC area%, even more preferably 2.0 GC area%, particularly preferably 1.5 GC area%, and most preferably 1.0 GC area%.

In the method for producing a cyclic diene-containing composition of the present invention, the content of a high-boiling-point byproduct contained in the cyclic diene-containing composition and having a higher boiling point than the cyclic diene is preferably controlled to a predetermined threshold (2) or more.

By controlling the content of the high-boiling-point byproduct to the predetermined threshold (2) or more, the cyclic diene-containing composition of the present invention is liquid at normal temperature (25°C), and thus has good fluidity and achieves excellent industrial handleability.

From the viewpoints described above, the predetermined threshold (2) is preferably 0.3 GC area%, more preferably 0.6 GC area%, even more preferably 1.0 GC area%, particularly preferably 3.0 GC area%, and most preferably 5.0 GC area%.

Furthermore, in the cyclic diene-containing composition of the present invention and the method for producing a cyclic diene-containing composition of the present invention, the content of a high-boiling-point byproduct contained in the cyclic diene-containing composition and having a higher boiling point than the cyclic diene is preferably controlled to a predetermined threshold (3) or less.

By controlling the content of the high-boiling-point byproduct to the predetermined threshold (3) or less, when the cyclic diene-containing composition is used as a starting material for an alicyclic aldehyde, the alicyclic aldehyde corresponding to the cyclic diene can be produced in a short reaction time and at a high yield.

From the viewpoints described above, the predetermined threshold (3) is preferably 25.0 GC area%, more preferably 20.0 GC area%, even more preferably 15.0 GC area%, particularly preferably 10.0 GC area%, and most preferably 8.0 GC area%.

The means for setting the content of the cyclic monoene contained in the cyclic diene-containing composition to the predetermined threshold (1) or less is not particularly limited, and an example thereof is a method of controlling distillation and purification conditions when a cyclic diene-containing composition containing the cyclic diene is produced by the distillation and purification of the hydrocarbon decomposition product. The details of the distillation and purification conditions will be described below.

Similarly to the above, the means for setting the content of the high-boiling-point byproduct contained in the cyclic diene-containing composition to the predetermined threshold (2) or more or the predetermined threshold (3) or less is not particularly limited, and an example thereof is a method of controlling distillation and purification conditions when a cyclic diene-containing composition containing the cyclic diene is produced by the distillation and purification of the hydrocarbon decomposition product. The details of the distillation and purification conditions will be described below.

### Hydrocarbon-Containing Composition

As the hydrocarbon-containing composition, naphtha, coal, and natural gas can be used.

Among these, naphtha is preferred from the viewpoint of excellent productivity and quality of the resulting cyclic diene-containing composition.

As the "naphtha" in the present invention, a known naphtha can be used. An embodiment of known naphtha can be a hydrocarbon-containing composition derived from crude oil having a boiling point range of approximately 30 to 230°C. Alternatively, another embodiment of known naphtha can be a hydrocarbon-containing composition containing a hydrocarbon having 5 or more and 12 or less carbons in an amount of 90 mass% or more per 100% of the total mass of the naphtha.

The "hydrocarbon having 5 or more and 12 or less carbons" described above is mainly an aromatic hydrocarbon such as benzene, toluene, xylene, ethylbenzene, or styrene; an aliphatic hydrocarbon such as normal pentane, 1-hexene, normal octane, 1-nonene, normal decane, and normal dodecane; or a naphthene such as methylcyclohexane or ethylcyclohexane.

### Ethylene Production Equipment

The ethylene production equipment in the present invention is production equipment for obtaining a C5 hydrocarbon fraction described below as one separated and purified fraction of a naphtha decomposition product. Specifically, the ethylene production equipment refers to equipment in which naphtha is thermally decomposed at a high temperature to form hydrogen, methane, ethane, ethylene, propane, propylene, hydrocarbons having 4 carbons such as butane or butadiene, hydrocarbons having 5 carbons, aromatic hydrocarbons such as benzene, and other heavy oils, and then these are separated and purified.

The ethylene production apparatus described above is not particularly limited and may be an apparatus having any configuration as long as the apparatus includes distillation equipment for a C5 hydrocarbon fraction and performs separation and purification of a naphtha decomposition product. An example thereof is a configuration including equipment performing the following steps (1) to (4) sequentially.
Step (1): Thermal decomposition step of thermally decomposing raw materials such as naphtha, coal, and natural gas in a decomposition furnace
Step (2): Quenching step of quenching and separating the obtained decomposition gas
Step (3): Compression step of compressing the quenched and separated decomposition gas
Step (4): Purification step of separating and purifying the compressed decomposition gas to produce ethylene and propylene which are main products

### C5 Hydrocarbon Fraction

The C5 hydrocarbon fraction in the present invention is a fraction containing a hydrocarbon having 5 carbons as a main component, the fraction being produced by separating a heavy oil component from a hydrocarbon decomposition product obtained by thermal decomposition (cracking) of a hydrocarbon-containing composition such as naphtha, and then further separating and removing hydrogen and a hydrocarbon having from 1 to 4 carbons.

Specific examples of the C5 hydrocarbon fraction include isoprene, isopentane, normal pentane, a monocyclic diene such as cyclopentadiene, and a mixture containing a C5 hydrocarbon as a main component. The C5 hydrocarbon fraction contains a small amount of a C4 hydrocarbon fraction and a small amount of a C6 hydrocarbon fraction in view of separation performance. Since the C5 hydrocarbon fraction contains cyclopentadiene, the cyclopentadiene is polymerized into dicyclopentadiene with the passage of time, and the C5 hydrocarbon fraction contains dicyclopentadiene.

An example of the composition of the C5 hydrocarbon fraction is shown in Table 1. Although it also varies depending on the type of the hydrocarbon-containing composition, such as naphtha, subjected to thermal decomposition, generally, the C5 hydrocarbon fraction contains cyclopentadiene and dicyclopentadiene in a total amount of 10 to 35 wt.%.

**[Table 1]**

| Component | wt.% |
|---|---|
| Hydrocarbon having 4 or less carbons | 0 to 10 |
| Hydrocarbons having 5 carbons (e.g., isopentane, n-pentane, pentene-l, pentene-2, isoprene, pentadiene) | 65 to 90 |
| Cyclopentadiene | 10 to 35 |
| Dicyclopentadiene | |
| Hydrocarbon having 6 or more carbons | 0 to 30 |

### Distillation and Purification of C5 Hydrocarbon Fraction

A single-stage or multi-stage distillation column can be used for distillation and purification of the C5 hydrocarbon fraction.

In particular, from the viewpoints of setting the content of the cyclic monoene contained in the cyclic diene-containing composition of the present invention to the predetermined threshold (1) or less, setting the content of the high-boiling-point byproduct to the predetermined threshold (2) or more, and setting the content of the high-boiling-point byproduct to the predetermined threshold (3) or less, preferably, a two-stage distillation column is used, and the number of theoretical plates of each distillation column and the reflux ratio thereof are controlled within the range described below.

In a case where a two-staged distillation column is used, specifically, when the number of theoretical plates is controlled to 10 to 20, and preferably 13 to 17, and the reflux ratio is controlled to a range of 20 to 30, and preferably 23 to 27 in the first-stage distillation column, the cyclic monoene can be efficiently separated and removed, and thus the predetermined threshold (1) can be efficiently controlled. When the number of theoretical plates is controlled to 10 to 20, and preferably 13 to 17, and the reflux ratio is controlled to a range of 1.0 to 1.5 in the second-stage distillation column, the high-boiling-point byproduct can be efficiently separated and removed to such an extent that it does not inhibit the hydroformylation reaction described below, and thus the predetermined threshold (2) and the predetermined threshold (3) can be efficiently controlled.

### Specific Embodiment of Method for Producing Cyclic Diene-Containing Composition

A specific embodiment of the method for producing a cyclic diene-containing composition of the present invention is a method of performing the following steps (I) to (IV) sequentially.
Step (I): A dimerization step in which a C5 hydrocarbon fraction described below obtained by thermal decomposition of a hydrocarbon-containing composition, such as naphtha, is introduced into a dimerization tank and heated, and a cyclic diene precursor such as cyclopentadiene contained in the C5 hydrocarbon fraction is dimerized, to form a cyclic diene, such as dicyclopentadiene, corresponding to the cyclic diene precursor.
Step (II): A recovery step in which the product derived from Step (I) is led to a recovery column and subjected to distillation, and then an unreacted component in Step (I) is recovered from the column top while a C5 fraction heavy composition that is rich in the cyclic diene, such as dicyclopentadiene, is taken out from the column bottom.
Step (III): A step of removing a low-boiling-point component, in which the C5 fraction heavy composition obtained from the column bottom in Step (II) is led to a low-boiling-point component removal column and distilled, and then a cyclic monoene having a low boiling point, such as vinylnorbornene, which is produced as a byproduct at the time of dimerization reaction in Step (I), and which has a lower boiling point than the cyclic diene, such as dicyclopentadiene, is removed from the column top while a fraction that is rich in a cyclic diene, such as dicyclopentadiene, is taken out from the column bottom.
   As the distillation conditions in Step (III), the distillation conditions of the first-stage distillation column described for the distillation and purification of the C5 hydrocarbon fraction described above can be employed.
Step (IV): A step of removing a high-boiling-point component, in which the fraction that is rich in a cyclic diene, such as dicyclopentadiene, obtained from the column bottom in Step (III) is led to a high-boiling-point component removal column and distilled, and then a high-boiling-point byproduct which has a high boiling point, such as analogous codimer, which has a higher boiling point than a cyclic diene, such as dicyclopentadiene, and which is produced as a byproduct at the time of the dimerization reaction in Step (I) is removed from the column bottom while the cyclic diene-containing composition of the present invention containing a high content of the cyclic diene, such as dicyclopentadiene, is taken out from the column top.

As the distillation conditions in Step (IV), the distillation conditions of the second-stage distillation column described for the distillation and purification of the C5 hydrocarbon fraction described above can be employed.

Steps (I) to (IV) will be described in more detail below.

### Step (I)

The C5 hydrocarbon fraction is first supplied to the dimerization step (I), and then the cyclic diene precursor, such as cyclopentadiene, contained in the C5 hydrocarbon fraction is dimerized, to form a cyclic diene corresponding to the cyclic diene precursor, such as dicyclopentadiene.

The conditions of the dimerization are not particularly limited and are appropriately selected based on the content of the cyclic diene precursor, such as cyclopentadiene, in the C5 hydrocarbon fraction and the like; however, generally, the dimerization temperature is set to a range of 50 to 110°C, the reaction time is set to a range of 1 to 6 hours, and 30 to 99 wt.% of the cyclic diene precursor, such as cyclopentadiene, contained in the C5 hydrocarbon fraction is dimerized.

### Step (II)

The product derived from the dimerization step (I) is then fed to the recovery step (II) of the unreacted component, and the unreacted C5 hydrocarbon fraction is separated and recovered. The unreacted C5 hydrocarbon fraction contains isoprene, piperylene (i.e., 1,3-pentadiene), and the like, and these are fed to separate purification processes as necessary.

For example, because the boiling point of dicyclopentadiene, which is a cyclic diene, is 170°C and the boiling points of other unreacted components are approximately from 30 to 50°C, the product derived from the dimerization step (I) is fed to a distillation column utilizing the boiling point differences of these in this recovery step (II). The unreacted C5 hydrocarbon fraction is recovered from the column top of the distillation column, and the C5 fraction heavy composition that is rich in a cyclic diene, such as dicyclopentadiene, is taken out from the column bottom.

As the distillation condition, the distillation is typically performed at normal pressure; however, distillation under reduced pressure or steam distillation is performed to recover larger amounts of the C5 hydrocarbon fraction and the C6 hydrocarbon fraction.

### Step (III)

The C5 fraction heavy composition taken out from the column bottom of the distillation column in Step (II) is, as necessary, liquefied by a condenser, and then fed to a step of removing a low-boiling-point component (III) (low-boiling-point component removal column) and subjected to distillation. The C5 fraction heavy composition fed to this Step (III) is managed so that the content of the cyclic diene, such as dicyclopentadiene, is typically in a range of 50 to 95 wt.%.

From the column top of the low-boiling-point component removal column, a low-boiling-point component contained in the C5 fraction heavy composition is removed while a fraction that is rich in a cyclic diene, such as dicyclopentadiene, is taken out from the column bottom.

The low-boiling-point component is, besides the C5 hydrocarbon fraction and the C6 hydrocarbon fraction that are not be recovered in Step (II), a cyclic monoene having a low boiling point such as vinylnorbornene, an analogous codimer such as propenylnorbornene obtained by heterodimerization of cyclopentadiene and piperylene, and the like which have lower boiling points than dicyclopentadiene and which are formed as byproducts at the time of dimerization reaction in Step (I).

Among these low-boiling-point components, a cyclic monoene such as vinylnorbornene, the C5 hydrocarbon fraction, and the C6 hydrocarbon fraction are easily removed, but an analogous codimer component such as propenylnorbornene is difficult to remove, and thus a 50- to 100-stage distillation column is typically required as the low-boiling-point component removal column.

The low-boiling-point component removal column is preferably controlled so that the content of a monocyclic diene such as cyclopentadiene is 0.5 GC area% or less and the content of a hydrocarbon having 5 carbons is 2.0 GC area% or less in the column bottom distillate obtained from the column bottom.

The distillation conditions in the low-boiling-point component removal column are typically controlled so that the degree of pressure reduction is 5 to 200 torr, and preferably from 10 to 50 torr, the column bottom temperature is 50 to 120°C, and preferably 80 to 110°C, the number of theoretical plates is 10 to 20, and preferably 13 to 17, and the reflux ratio is in a range of 20 to 30, and preferably 23 to 27. Under these conditions, 90 wt.% or more of a cyclic monoene such as vinylnorbornene and an analogous codimer contained in the C5 fraction heavy composition obtained from the column bottom of the low-boiling-point component removal column in Step (II) can be efficiently separated and removed from the column bottom distillate obtained from the column bottom of the low-boiling-point component removal column. Specifically, the content of a monocyclic diene such as cyclopentadiene can be adjusted to 0.5 GC area% or less and the content of a hydrocarbon having 5 carbons can be adjusted to 2.0 GC area% or less in the column bottom distillate.

### Step (IV)

The fraction (column bottom liquid) that is rich in a cyclic diene, such as dicyclopentadiene, taken out from the column bottom of the low-boiling-point component removal column in Step (III) of removing the low-boiling-point component is fed to Step (IV) of removing a high-boiling-point component (high-boiling-point component removal column) and subjected to distillation.

The high-boiling-point component removed in this Step (IV) is a high-boiling-point byproduct, such as an analogous codimer having a high boiling point, such as methylbicyclononadiene, obtained by dimerization of cyclopentadiene and isoprene, the analogous codimer having a higher boiling point than the cyclic diene such as dicyclopentadiene and being produced as a byproduct at the time of dimerization reaction in Step (I). In a case where low polymers, such as trimers of cyclopentadiene are present, these low polymers are also removed.

The distillation conditions in the high-boiling-point component removal column in Step (IV), especially the distillation temperature, affect thermal decomposition of dicyclopentadiene and significantly affect the purity of the final product. Thus, attention is preferably paid to obtain a target cyclic diene-containing composition.

The high-boiling-point component removal column is preferably a packed column to make pressure drop in the column as small as possible, and typically, the high-boiling-point component removal column is controlled so that the degree of pressure reduction is 5 to 100 torr, and preferably from 10 to 50 torr, the column bottom temperature is 50 to 120°C, and preferably 70 to 110°C, the number of theoretical plates is 10 to 20, and preferably 13 to 17, and the reflux ratio is in a range of 1.0 to 1.5. Under these conditions, in the cyclic diene-containing composition of the present invention obtained from the column top of the high-boiling-point component removal column, a high-boiling-point byproduct contained in a column bottom distillate obtained from the column bottom of the low-boiling-point component removal column in Step (III) can be efficiently separated and removed to such an extent that it does not inhibit a hydroformylation reaction described below.

Specifically, in the cyclic diene-containing composition obtained from the column top of the high-boiling-point component removal column, preferably, the content of the cyclic diene such as dicyclopentadiene can be controlled to 99.5 GC area% or less and the content of the cyclic monoene can be controlled to the aforementioned threshold (1) or less, e.g., 14.0 GC area% or less, and preferably the content of the vinyl group-containing cyclic monoene having 9 carbons such as vinylnorbornene can be controlled to 3.0 GC area% or less, more preferably, the content of the high-boiling-point byproduct can be controlled to 0.3 GC area% or more, and even more preferably, the content of the high-boiling-point byproduct can be controlled to the aforementioned threshold (3) or less, e.g., 25 GC area% or less.

The method of controlling the composition of the cyclic diene-containing composition obtained from the column top of the high-boiling-point component removal column to the aforementioned composition is not particularly limited, and the method is, for example, a method of designing and manufacturing a device so that the column bottom temperature of the high-boiling-point component removal column is in a range of 50 to 120°C and the degree of pressure reduction is in a range of 5 to 100 torr, the number of theoretical plates is in a range of 10 to 20, and the reflux ratio is in a range of 1.0 to 1.5, and the retention time of a distillation column feed liquid in the column is 15 minutes or shorter, and preferably 10 minutes or longer and 15 minutes or shorter.

When the column bottom temperature of the high-boiling-point component removal column is lower than 50°C, the content of a C5 hydrocarbon, including a monocyclic diene such as cyclopentadiene, and a cyclic monoene in the column bottom distillate is increased. When the column bottom temperature is higher than 120°C, the content of the high-boiling-point byproduct, such as tricyclopentadiene, in the column bottom distillate is increased. In both of the cases, the content of dicyclopentadiene in the column bottom distillate is reduced.

When the number of theoretical plates is controlled to a range of 10 to 20 and the reflux ratio is controlled to a range of 1.0 to 1.5 in the high-boiling-point component removal column, in the cyclic diene-containing composition of the present invention obtained from the column top of the high-boiling-point component removal column, the high-boiling-point byproduct can be separated and removed to such an extent that it does not inhibit the following hydroformylation reaction.

When the retention time of the distillation column feed liquid in the column is longer than 15 minutes, the content of the high-boiling-point byproduct, such as tricyclopentadiene, in the column bottom distillate is increased. When the retention time of the distillation column feed liquid in the column is shorter than 10 minutes, the column bottom portion of the distillation column becomes smaller, and an instrument for liquid surface measurement becomes difficult to install. Furthermore, the liquid amount held in the column bottom is decreased, and stable operation may be impaired when the liquid inside the column bottom becomes empty due to typical operation change of the distillation column.

Examples of the specific method of achieving the aforementioned retention time include designing the inner diameter of the column bottom of the distillation column in a size that matches the amount of the column bottom distillate component.

The pressure of the distillation column described above may be controlled by introducing, into the distillation column, a gas that does not contain a hydrocarbon having 5 or more carbons (pressure controlling gas) present in the treatment equipment of the naphtha decomposition product described above.

### One Embodiment of Production Equipment of Cyclic Diene-Containing Composition

Next, one embodiment of production equipment of the cyclic diene-containing composition of the present invention will be described with reference to a drawing illustrating the equipment.

As illustrated in FIG. 1, the C5 hydrocarbon fraction obtained by thermal decomposition of a hydrocarbon-containing composition, such as naphtha, is first supplied to a dimerization tank 1 through a tube 10. In this tank, a cyclic diene precursor such as cyclopentadiene is dimerized, to form a cyclic diene such as dicyclopentadiene corresponding to the cyclic diene precursor [dimerization step (I)]. After the dimerization reaction, the content of the dimerization tank 1 is fed to a distillation column 2 [recovery step (II) of an unreacted component] through a tube 11 and subjected to distillation.

In the distillation column 2, the unreacted C5 hydrocarbon fraction is removed from the column top of the distillation column 2 through a tube 13, a column bottom liquid that is rich in the cyclic diene such as dicyclopentadiene (C5 fraction heavy composition) is fed from the column bottom of the distillation column 2 through a tube 12 to a low-boiling-point component removal column 3 [step (III) of removing a low-boiling-point component] and subjected to distillation.

In the low-boiling-point component removal column 3, the cyclic monoene such as vinylnorbornene, the C5 hydrocarbon fraction, the C6 hydrocarbon fraction, and the analogous codimer, which have a lower boiling point than the cyclic diene such as dicyclopentadiene, are removed from the column top of the low-boiling-point component removal column 3 through a tube 15, and a fraction that is rich in the cyclic diene such as dicyclopentadiene is fed from the column bottom of the low-boiling-point component removal column 3 through the tube 14 to a high-boiling-point component removal column 4, i.e., the last column [step (IV) of removing high-boiling-point component] and subjected to distillation.

In the high-boiling-point component removal column 4, the high-boiling-point analogous codimer having a higher boiling point than the cyclic diene such as dicyclopentadiene and the remaining low polymers are removed from the column bottom of the high-boiling-point component removal column 4 through a tube 16, and the cyclic diene-containing composition containing a high content of the cyclic diene such as dicyclopentadiene is obtained from the column top of the high-boiling-point component removal column 4 through a tube 17.

The order of performing the aforementioned Step (I) to Step (IV), especially Step (II) to Step (IV), is not particularly limited, and the order of performing these steps can be changed as long as the effects of the present invention are not impaired.

### Method for Producing Aldehyde

The method for producing an aldehyde, which is the second embodiment of the present invention, will be described below.

### Embodiment 2-1

The embodiment 2-1 of the method for producing an aldehyde of the present invention is a method for producing an aldehyde corresponding to a cyclic diene by subjecting the cyclic diene in a cyclic diene-containing composition described below to a hydroformylation reaction, the method including controlling the content of a cyclic monoene contained in the cyclic diene-containing composition to a predetermined threshold (1) or less.

By controlling the content of the cyclic monoene contained in the cyclic diene-containing composition to the predetermined threshold (1) or less, an alicyclic aldehyde corresponding to the cyclic diene can be produced in a short reaction time and at a high yield.

From the viewpoints described above, the predetermined threshold (1) is preferably 14.0% or less, more preferably 12.0% or less, even more preferably 10.0% or less, particularly preferably 8.0% or less, and most preferably 7.0% or less, per 100% of the total GC area of the cyclic diene-containing composition.

In a case where the cyclic monoene is a vinyl group-containing cyclic monoene having 9 carbons, such as vinylnorbornene, from the viewpoints described above, the predetermined threshold (1) is preferably 3.0 GC area%, more preferably 2.5 GC area%, even more preferably 2.0 GC area%, particularly preferably 1.5 GC area%, and most preferably 1.0 GC area%.

The details of the cyclic monoene are as described above.

Furthermore, in the method for producing an aldehyde of the present invention, the method preferably controls the content of a high-boiling-point byproduct having a higher boiling point than the cyclic diene contained in the cyclic diene-containing composition to a predetermined threshold (2) or more.

By controlling the content of the high-boiling-point byproduct to the predetermined threshold (2) or more, superior fluidity of the cyclic diene-containing composition of the present invention at normal temperature (25°C) can be achieved.

From the viewpoints described above, the predetermined threshold (2) is preferably 0.3 GC area%, more preferably 0.6 GC area%, even more preferably 1.0 GC area%, particularly preferably 3.0 GC area%, and most preferably 5.0 GC area%.

Furthermore, in the method for producing an aldehyde of the present invention, the method preferably controls the content of a high-boiling-point byproduct having a higher boiling point than the cyclic diene contained in the cyclic diene-containing composition to a predetermined threshold (3) or less.

By controlling the content of the high-boiling-point byproduct to the predetermined threshold (3) or less, the alicyclic aldehyde corresponding to the cyclic diene can be produced in a short reaction time and at a high yield.

From the viewpoints described above, the predetermined threshold (3) is preferably 25.0 GC area%, more preferably 20.0 GC area%, even more preferably 15.0 GC area%, particularly preferably 10.0 GC area%, and most preferably 8.0 GC area%.

The details of the high-boiling-point byproduct are as described above.

In the method for producing an aldehyde according to the embodiment 2-1 of the present invention, the methods of controlling the predetermined threshold (1), the predetermined threshold (2), and the predetermined threshold (3) are not particularly limited. Examples thereof include a method of controlling the distillation and purification conditions when the cyclic diene-containing composition is produced by distillation and purification of the hydrocarbon decomposition product obtained by thermal decomposition of the hydrocarbon-containing composition, such as naphtha, as described above.

### Embodiment 2-2

The method for producing an aldehyde according to the embodiment 2-2 of the present invention is a method for producing an aldehyde, the method including producing an aldehyde corresponding to the cyclic diene by subjecting the cyclic diene-containing composition, which is the first embodiment of the present invention, to a hydroformylation reaction.

### Embodiment 2-3

The method for producing an aldehyde according to the embodiment 2-3 of the present invention is a method for producing an aldehyde, the method including producing a cyclic diene-containing composition (hereinafter may also be referred to as "the cyclic diene-containing composition of the present invention") by the method for producing a cyclic diene-containing composition, which is the third embodiment of the present invention, and producing a corresponding aldehyde from a cyclic diene contained in the cyclic diene-containing composition.

The methods for producing an aldehyde according to the embodiments 2-1, 2-2, and 2-3 of the present invention may be simply and collectively referred to as "the method for producing an aldehyde of the present invention" hereinafter.

In the method for producing an aldehyde of the present invention, the method of producing an aldehyde corresponding to a cyclic diene from the cyclic diene contained in a cyclic diene-containing composition by the hydroformylation reaction is not particularly limited and can be performed in accordance with an ordinary method.

For example, in accordance with the method described in JP 2001-10999 A, in a hydroformylation reaction solvent containing a hydrocarbon compound, in the coexistence with a catalyst containing a rhodium compound and an organophosphorus compound, the cyclic diene contained in the cyclic diene-containing composition of the present invention can be subjected to hydroformylation by using hydrogen and carbon monoxide, to produce an aldehyde corresponding to the cyclic diene.

Specifically, when the cyclic diene in the cyclic diene-containing composition of the present invention is dicyclopentadiene, in accordance with the method described in JP 2001-10999 A, in a hydroformylation reaction solvent containing a hydrocarbon compound, in the coexistence with a catalyst containing a rhodium compound and an organophosphorus compound, dicyclopentadiene contained in the cyclic diene-containing composition can be subjected to hydroformylation by using hydrogen and carbon monoxide as described below in Reaction Formula (I), to produce tricyclodecane dicarbaldehyde.

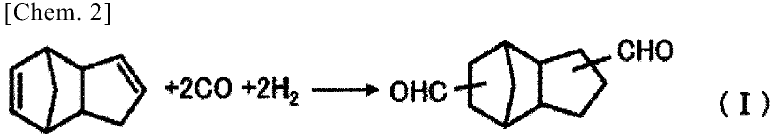

The rhodium compound used in this hydroformylation step may have any precursor form as long as it forms a complex with an organophosphorus compound and exhibits hydroformylation activity in the presence of hydrogen and carbon monoxide.

As the rhodium compound, a rhodium metal hydride carbonyl phosphorus complex having catalytic activity may be formed in a reaction vessel by introducing a catalyst precursor substance, such as Rh(acac)(CO)₂, Rh₂O₃, Rhs(CO)₁₂, Rh₆(CO)₁₆, or Rh(NO₃)₃, together with an organophosphorus compound into a reaction mixture. Alternatively, a rhodium metal hydride carbonyl phosphorus complex catalyst may be prepared in advance and may be introduced into a reaction vessel.

In a specific preferred example of the present invention, Rh(acac)(CO)₂ is used as a rhodium precursor substance and reacted with an organophosphorus compound in the presence of a solvent and then introduced into a reactor together with an excessive amount of a free-form organophosphorus compound, to form a rhodium-organophosphorus complex catalyst having catalytic activity.

Examples of the organophosphorus compound forming a catalyst of a hydroformylation reaction together with a rhodium compound include known phosphites and known phosphines.

Among the phosphites, preferred is a compound represented by General Formula P (-OR¹¹)(-OR¹²)(-OR¹³) (wherein R¹¹, R¹², and R¹³ each represent an optionally substituted aryl group or alkyl group). Specific examples of R¹¹, R¹², and R¹³ include an aryl group, such as a phenyl group or a naphthyl group, that may be substituted with a methyl group, an ethyl group, an isopropyl group, an n-butyl group, a t-butyl group, a methoxy group, or the like; an aliphatic alkyl group such as a methyl group, an ethyl group, an isopropyl group, an n-butyl group, or a t-butyl group; and an alicyclic alkyl group, such as a cyclopentyl group or a cyclohexyl group, that may be substituted with a lower alkyl group such as a methyl group, an ethyl group, an isopropyl group, an n-butyl group, or a t-butyl group.

Specific examples of the preferred phosphite include tris(2-t-butylphenyl) phosphite, tris(3-methyl-6-t-butylphenyl) phosphite, tris(3-methoxy-6-t-butylphenyl) phosphite, tris(2,4-di-t-butylphenyl) phosphite, and di(2-t-butylphenyl) (t-butyl)phosphite. The phosphite is not limited to these. These phosphites may be used alone or in combination of two or more types.

As the phosphine, a sterically-hindered alkyl phosphine is effective for the hydroformylation reaction of dicyclopentadiene. Specific examples thereof include tricyclopropylphosphine, tricyclobutylphosphine, tricyclopentylphosphine, tricyclohexylphosphine, tricycloheptylphosphine, and tricyclooctylphosphine.

The phosphine is not limited to these. These phosphines may be used alone or in combination of two or more types.

The amount of the organophosphorus compound used is not particularly limited and can be appropriately set according to known techniques by a person skilled in the art.

The amount of the rhodium catalyst used is also not particularly limited and can be appropriately set according to known techniques by a person skilled in the art.

The hydroformylation reaction of dicyclopentadiene can be performed without using any solvent; however, the hydroformylation reaction can be more suitably performed by using an organic solvent that is inactive for the reaction.

The temperature and pressure of the hydroformylation reaction of dicyclopentadiene are not particularly limited and can be appropriately set according to known techniques by a person skilled in the art. The reaction temperature is generally from 40 to 160°C, and preferably from 80 to 140°C, and the reaction pressure is generally from 1 to 15 MPa.

The molar ratio of hydrogen and carbon monoxide in the hydrogen/carbon monoxide gas mixture used in the reaction is not particularly limited and can be appropriately set according to known techniques by a person skilled in the art. Generally, as an introduction gas composition, the molar ratio of hydrogen to carbon monoxide (hydrogen/carbon monoxide) can be set to be in a range of 0.2 to 5.0.

As the reaction method of the hydroformylation, a continuous feeding method is employed. In the continuous feeding method, the hydroformylation is performed while the cyclic diene-containing composition of the present invention alone that serves as a raw material or a mixed solution of the cyclic diene-containing composition of the present invention and a solvent is supplied to a reactor, in which the rhodium-organophosphorus complex catalyst, the solvent, and the gas mixture of hydrogen and carbon monoxide are present. By using this method, dicyclopentadiene, which is the cyclic diene in the cyclic diene-containing composition of the present invention, is thermally decomposed in the reactor, and formation of cyclopentadiene that inhibits the hydroformylation reaction can be reduced, and thus good reaction rate and yield can be maintained. To maintain the fluidity of the cyclic diene-containing composition of the present invention, dilution is preferably performed with the solvent described above, and the diluted cyclic diene-containing composition is preferably supplied to the reactor at a temperature that does not allow depolymerization of the composition and that does not allow generation of cyclopentadiene.

### Extraction of Tricyclodecane Dicarbaldehyde

The method for extraction and separation of tricyclodecane dicarbaldehyde, which is a product, from the reaction product solution after the completion of the hydroformylation reaction is not particularly limited and can be appropriately set according to known techniques by a person skilled in the art. For example, an extraction and separation method described in JP 2001-10999 A can be used.

### Method for Producing Alcohol

The method for producing an alcohol of the present invention is a method for producing an alcohol, the method including producing an aldehyde by the method for producing an aldehyde of the present invention described above, and producing a corresponding alcohol from the aldehyde.

The method for producing an alcohol corresponding to the aldehyde is not particularly limited and can be performed in accordance with an ordinary method. For example, according to the method described in JP 2001-10999 A, an alcohol can be produced by subjecting the aldehyde obtained by the method for producing an aldehyde of the present invention as is to a known hydrogenation reaction, or by subjecting the obtained aldehyde to dimerization and then to a known hydrogenation reaction.

Specifically, when the cyclic diene in the cyclic diene-containing composition of the present invention is dicyclopentadiene, dicyclopentadiene contained in the cyclic diene-containing composition is subjected to hydroformylation to produce tricyclodecane dicarbaldehyde, then the obtained tricyclodecane dicarbaldehyde is subjected to a hydrogenation reaction in the presence of a hydrogenation catalyst and hydrogen. Thus, tricyclo[5.2.1.0(2,6)]decanedimethanol can be produced.

As the hydrogenation catalyst used in the hydrogenation reaction, a known solid catalyst in which a metal such as Ru, Ni, Cr, or Cu is carried on a carrier can be used. A Ru-based catalyst is preferred.

The conditions of the hydrogenation reaction generally include a temperature of 60 to 200°C and a hydrogen pressure of approximately 0.1 to 20 MPaG.

### Examples

The present invention will be more specifically described below with reference to Reference Experimental Examples and Experimental Examples (hereinafter simply referred to as "Experimental Examples") in place of Examples and Comparative Examples. The present invention is not limited to the following Experimental Examples as long as the Experimental Examples do not depart from the gist of the present invention.

The following experimental examples are simply examples and are not intended to limit any embodiments described in the present specification. The following experimental examples do not limit the present invention.

Values of various production conditions and evaluation results in the following experimental examples have significance as preferable values of upper limits or lower limits in the embodiments of the present invention. A preferable range in the present invention may be a range defined by a combination of the value of an upper limit or lower limit described above and a value in the following experimental examples or values between the experimental examples.

The compounds used in the following experimental examples are as follows.
Commercially available DCPD: (product name: High-Purity Dicyclopentadiene, available from Maruzen Petrochemical Co., Ltd.)
Acetylacetonate dicarbonyl rhodium (product name: Rh(acac)(CO)₂, available from N.E. Chemcat Corporation)
Ruthenium-supported catalyst (Ru-supported carbon; dry-base Ru content: 5%; water content: 56%) (product name: Ru/C, available from N.E. Chemcat Corporation)
Tris(2,4-di-tert-butylphenyl) phosphite (product name: DBPO, available from Tokyo Chemical Industry Co., Ltd.)
Methylcyclohexane (product name: Methylcyclohexane, available from FUJIFILM Wako Pure Chemical Corporation)
Methanol (product name: Methanol, available from FUJIFILM Wako Pure Chemical Corporation)

### Reference Experimental Example 1

### Production of Heavy Oil by Thermal Decomposition of Naphtha

Using commercially available naphtha as a hydrocarbon-containing composition and a naphtha decomposition plant manufactured in-house, a hydrocarbon decomposition product was obtained by subjecting the naphtha to thermal decomposition by the following procedure with reference to a thermal decomposition method of naphtha described in JP 7-39354.

The obtained hydrocarbon decomposition product was supplied to a distillation column, and a C5 hydrocarbon fraction containing a hydrocarbon having 5 carbons as a main component was recovered.

Subsequently, the obtained C5 hydrocarbon fraction was supplied to a reaction distillation column, and while cyclopentadiene contained in the C5 hydrocarbon fraction was dimerized and converted into dicyclopentadiene, separation and purification were performed under the following conditions. Thus, a C5 fraction heavy composition containing dicyclopentadiene, i.e., a cyclic diene was recovered from the column bottom of the distillation column.

The composition of the obtained C5 fraction heavy composition was analyzed by using a gas chromatograph (GC) and a gas chromatography total area method under the following GC measurement conditions. The results are as follows. The obtained gas chromatogram is illustrated in FIG. 6.

The compositional proportion of each component in the gas chromatography total area method was calculated as an area content (unit: GC area%) of each peak component when the total area of GC peaks of all products in the gas chromatogram was taken as 100%.

### GC Measurement Conditions

GC apparatus: GC-2025 (high-performance general-purpose gas chromatograph, available from Shimadzu Corporation)
Detector: flame ionization detector (FID)
Carrier gas: helium (column flow rate: 1.65 mL/min)
Column: Capillary Column DB-1 (available from Agilent Technologies; size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

### Composition of C5 Fraction Heavy Composition

Component (1) 2.2 GC area%
Vinylnorbornene 0.6 GC area%
Component (2) 1.4 GC area%
Isopropenylnorbornene 6.6 GC area%
DCPD 75.9 GC area%
High-boiling-point byproduct 13.3 GC area%

"Component (1)" is a generic term of a plurality of components observed as a plurality of GC peaks in a temperature condition region lower than the boiling point of vinylnorbornene (retention time: 2.5 minutes or more and less than 11.2 minutes) in the gas chromatogram.

"Isopropenylnorbornene" is isopropenyl-2-norbornene (retention time: 14.0 minutes or more and less than 14.4 minutes).

"Component (2)" is a generic term of a plurality of components observed as a plurality of GC peaks in a temperature condition region higher than the boiling point of vinylnorbornene and lower than the boiling point of isopropenylnorbornene (retention time: 11.6 minutes or more and less than 14.0 minutes) in the gas chromatogram.

"Vinylnorbornene" is 5-vinyl-2-norbornene (retention time: 11.2 minutes or more and less than 11.6 minutes).

"DCPD" is dicyclopentadiene (retention time: 14.4 minutes or more and less than 15.0 minutes).

"High-boiling-point byproduct" is a component for which GC peaks were observed in a temperature condition region higher than the boiling point of dicyclopentadiene (retention time: 15.0 minutes or more and 30.0 minutes or less) in the gas chromatogram.

The cyclic monoene observed in the C5 fraction heavy composition was two types, i.e., vinylnorbornene and isopropenylnorbornene.

### Reference Experimental Example 2

### Distillation and Purification of Heavy Oil

1878 g of the C5 fraction heavy composition obtained in Reference Experimental Example 1 was charged in a glass-made simple distillation column, and subjected to fractional distillation operation under the following fractional distillation conditions, and fractions of distillate (approximately 100 g each) were taken from the column top of the distillation column and used as fractions 1 to 14 in the order of distillation.

### Fractional Distillation Conditions

Column bottom temperature: 40 to 113°C
Column top temperature: 24 to 80°C
Pressure: 1 to 10 kPa (7.5 to 75 torr)
Retention time in column of distillation column feed liquid: approximately 19 hours

The compositions of the fractions 1 to 14 and the commercially available DCPD were analyzed by using a gas chromatograph (GC) and a gas chromatography total area method under the following GC measurement conditions. The results are shown in Table 2.

The compositional proportion of each component in the gas chromatography total area method was calculated as an area content (unit: GC area%) of each peak component when the total area of GC peaks of all products observed in a temperature condition region of a retention time of 2.5 to 30 minutes in the gas chromatogram was taken as 100%.

For the fraction 2, the obtained gas chromatogram is illustrated in FIG. 7. The peak observed at a retention time of 2.2 minutes in the gas chromatogram is a peak of the solvent used for the GC measurement.

### GC Measurement Conditions

GC apparatus: GC-2025 (high-performance general-purpose gas chromatograph, available from Shimadzu Corporation)
Detector: flame ionization detector (FID)
Carrier gas: helium (column flow rate: 1.65 mL/min)
Column: Capillary Column DB-1 (available from Agilent Technologies; size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

In the obtained gas chromatogram, the retention time of each of the components was as follows. Furthermore, "component (1)", "isopropenylnorbornene", "component (2)", "vinylnorbornene", and "high-boiling-point byproduct" are respectively synonymous with the "component (1)", "isopropenylnorbornene", "component (2)", "vinylnorbornene", and "high-boiling-point byproduct" in the gas chromatogram measurement for the C5 fraction heavy composition.

Component (1) 2.5 minutes or more and less than 11.2 minutes
Vinylnorbornene 11.2 minutes or more and less than 11.6 minutes
Component (2) 11.6 minutes or more and less than 14.0 minutes
Isopropenylnorbornene 14.0 minutes or more and less than 14.4 minutes
DCPD 14.4 minutes or more and less than 15.0 minutes
High-boiling-point byproduct 15.0 minutes or more and 30.0 minutes or less

The cyclic monoene observed in the fractions 1 to 6 was two types, i.e., vinylnorbornene and isopropenylnorbornene. The cyclic monoene observed in the fractions 7 to 14 and the commercially available DCPD was one type, i.e., isopropenylnorbornene.

Properties at normal temperature (25°C) of the fractions 1 to 14 and the commercially available DCPD were visually observed. The results are shown in Table 3.

**[Table 2]**

| | Component (1) | Component (2) | Cyclic monoene | | | DCPD | High-boiling-point byproduct |
|---|---|---|---|---|---|---|---|
| | | | (a) Vinylnorbornene | (b) Isopropenylnorbornene | (a)+(b) | | |
| | GC area% | GC area% | GC area% | GC area% | GC area% | GC area% | GC area% |
| Fraction 1 | 2.0 | 3.6 | 3.1 | 9.0 | 12.1 | 76.9 | 5.4 |
| Fraction 2 | 0.6 | 3.8 | 2.7 | 9.8 | 12.5 | 78.1 | 5.0 |
| Fraction 3 | 0.1 | 3.2 | 1.8 | 9.5 | 11.3 | 80.1 | 5.3 |
| Fraction 4 | 0.1 | 2.1 | 0.7 | 8.7 | 9.4 | 82.7 | 5.7 |
| Fraction 5 | 0.0 | 1.2 | 0.2 | 7.4 | 7.6 | 84.3 | 6.9 |
| Fraction 6 | 0.0 | 1.0 | 0.1 | 7.6 | 7.6 | 85.1 | 6.3 |
| Fraction 7 | 0.0 | 0.6 | 0.0 | 6.3 | 6.3 | 85.2 | 7.9 |
| Fraction 8 | 0.0 | 0.5 | 0.0 | 6.1 | 6.1 | 85.7 | 7.7 |
| Fraction 9 | 0.0 | 0.4 | 0.0 | 5.2 | 5.2 | 85.6 | 8.8 |
| Fraction 10 | 0.0 | 0.2 | 0.0 | 4.5 | 4.5 | 86.0 | 9.2 |
| Fraction 11 | 0.0 | 0.1 | 0.0 | 3.3 | 3.3 | 87.1 | 9.5 |
| Fraction 12 | 0.0 | 0.0 | 0.0 | 3.0 | 3.0 | 85.4 | 11.5 |
| Fraction 13 | 0.0 | 0.0 | 0.0 | 2.0 | 2.0 | 81.7 | 16.2 |
| Fraction 14 | 0.0 | 0.0 | 0.0 | 0.3 | 0.3 | 72.9 | 26.8 |
| Commercially available DCPD | 0.0 | 0.0 | 0.0 | 0.1 | 0.1 | 99.7 | 0.2 |

### Experimental Example 2

### Hydroformylation Reaction

In a stainless steel autoclave reactor having an internal volume of 100 mL, in an nitrogen atmosphere, 14.0 g of the fraction 2 as a raw material compound of a hydroformylation reaction catalyst, 7.1 mg (0.0275 mmol) of Rh(acac)(CO)₂ as a catalyst, 558.3 mg (0.863 mmol) of tris(2,4-di-t-butylphenyl) phosphite, and 11.2 g of methylcyclohexane as an organic solvent were charged, and then, while stirring was performed at 1500 rpm, the temperature of the reaction liquid in the reactor was increased to 70°C. Subsequently, a gas mixture of hydrogen and carbon monoxide (hydrogen:carbon monoxide = 1:1 (molar ratio)) was injected rapidly through a gas introduction valve so that the pressure in the reactor became 5 MPaG. While this pressure was maintained, the temperature of the reaction liquid was increased to 100°C, and the reaction was performed until hydrogen and carbon monoxide were not consumed by the reaction. The time at which hydrogen and carbon monoxide were not consumed by the reaction was taken as a reaction completion time of the hydroformylation reaction and used as an indicator of reaction efficiency. The gas mixture consumed for the reaction during the reaction was automatically introduced into the autoclave through an automatic pressure regulation valve, and the reaction was performed while the pressure in the reactor was constantly maintained at 5 MPaG.

After completion of the reaction, the reaction liquid in the reactor was cooled to room temperature, the remained gas in the reactor was discharged, and 31.3 g of a hydroformylation reaction product liquid was obtained.

### Evaluation of Hydroformylation Reaction

For the hydroformylation reaction product liquid described above, by using a gas chromatograph (GC) and a gas chromatography total area method under the following GC measurement conditions, the content (unit: GC area%) of dicyclopentadiene contained in the fraction 2 and the contents (unit: GC area%) of dicyclopentadiene (DCPD) contained in the hydroformylation reaction product liquid and tricyclodecane dicarbaldehyde (TCDDD), i.e., the product were determined, and the DCPD conversion rate (%) and the TCDDD yield (%) were calculated. The DCPD conversion rate was 99.1%, and the TCDDD yield was 76.9%. The evaluation results are shown in Table 3.

### GC Measurement Conditions

GC apparatus: GC-2025 (high-performance general-purpose gas chromatograph, available from Shimadzu Corporation)
Detector: flame ionization detector (FID)
Carrier gas: helium (column flow rate: 1.23 mL/min)
Column: Capillary Column DB-1 (available from Agilent Technologies; size: length 30 m × inner diameter 0.25 mm, film thickness 1.00 µm)
Column temperature: 120°C (retention time: none) → temperature increase at 10°C/min → 300°C (retention time: 12 minutes)
Inlet temperature: 200°C
Detector temperature: 300°C
Sample amount: 0.3 µL (split ratio: 1/30)

### Production of Alcohol

### Extraction Operation

To 31.3 g of the obtained hydroformylation reaction product liquid were added 5.0 g of methanol and 9.4 g of water, and the mixture was stirred in an nitrogen atmosphere for 10 minutes. Thereafter, the mixture was allowed to stand for 10 minutes to separate into two phases, and extraction operation was performed. To the obtained lower phase (a1) was added 1.5 g of methylcyclohexane, and the mixture was stirred for 30 minutes. Thereafter, the mixture was allowed to stand for 10 minutes to separate into two phases to perform extraction operation, and 34.4 g of a lower phase (a2) was obtained.

The composition of the obtained lower phase (a2) was analyzed by gas chromatography. The results were as follows: 47 mass% of tricyclodecane dicarbaldehyde, 27 mass% of methanol, 14 mass% or water, 7 mass% or methylcyclohexane, and 5 mass% of other components.

### Hydrogenation Reduction Reaction

In an autoclave reactor having an internal volume of 0.2 L, 34.4 g of the lower phase (a2) obtained by the extraction operation described above and 0.1 g of a ruthenium-supported catalyst were charged and, while stirring was performed at 120 rpm, the temperature of the reaction liquid in the reactor was increased to 160°C. Subsequently, a hydrogen gas was injected through a gas introduction valve so that the pressure in the reactor was 5 MPaG and, while the pressure and temperature of the reaction liquid were maintained, reaction was allowed for 2.3 hours. The hydrogen gas in an amount consumed for the reaction during the reaction was continuously introduced into the reactor while the pressure in the reactor was maintained at 5 MPaG.

After completion of the reaction, the reaction liquid in the reactor was cooled to room temperature, the remained gas in the reactor was discharged, the ruthenium-supported catalyst was separated by filtration using a 5 µm filter, and 34.6 g of a reaction product liquid was obtained. The amount of tricyclodecane dicarbaldehyde, i.e., the raw material compound contained in the reaction liquid before the reaction and the amount of tricyclo[5.2.1.0(2,6)]decanedimethanol (hereinafter, referred to as "TCDDM"), i.e., a product in the reaction product liquid after the reaction were analyzed by gas chromatography. As a result, the TCDDM yield was 94%.

### Experimental Example 1 and Experimental Examples 3 to 15

The hydroformylation reaction of the raw material compound was performed under the same conditions as in Experimental Example 2 except for using the fraction or the commercially available DPCD shown in Table 3 in place of the fraction 2 as the raw material compound in the hydroformylation reaction of Experimental Example 2. The evaluation results are shown in Table 3.

**[Table 3]**

| | Raw material compound | Properties | Hydroformylation reaction completion time | DCPD conversion rate | TCDDD yield |
|---|---|---|---|---|---|
| | | | Hour | GC area% | GC area% |
| Experimental Example 1 | Fraction 1 | Liquid | 3.6 | 98.9 | 76.5 |
| Experimental Example 2 | Fraction 2 | Liquid | 3.0 | 99.1 | 76.9 |
| Experimental Example 3 | Fraction 3 | Liquid | 2.6 | 98.3 | 76.8 |
| Experimental Example 4 | Fraction 4 | Liquid | 2.4 | 99.3 | 81.6 |
| Experimental Example 5 | Fraction 5 | Liquid | 2.4 | 99.2 | 82.1 |
| Experimental Example 6 | Fraction 6 | Liquid | 2.3 | 99.1 | 80.7 |
| Experimental Example 7 | Fraction 7 | Liquid | 2.2 | 98.8 | 83.2 |
| Experimental Example 8 | Fraction 8 | Liquid | 2.3 | 98.8 | 83.7 |
| Experimental Example 9 | Fraction 9 | Liquid | 2.4 | 99.1 | 82.8 |
| Experimental Example 10 | Fraction 10 | Liquid | 2.3 | 99.0 | 84.6 |
| Experimental Example 11 | Fraction 11 | Liquid | 2.5 | 99.1 | 88.1 |
| Experimental Example 12 | Fraction 12 | Liquid | 2.2 | 98.9 | 87.0 |
| Experimental Example 13 | Fraction 13 | Liquid | 2.8 | 99.0 | 86.9 |
| Experimental Example 14 | Fraction 14 | Liquid | 4.0 | 98.9 | 77.9 |
| Experimental Example 15 | Commercially available DCPD | Solid | 2.2 | 100 | 97.8 |

As shown in Table 2, the total value ((a) + (b)) of the contents (GC area%) of (a) vinylnorbornene and (b) isopropenylnorbornene was used as the content (GC area%) of the cyclic monoene.

For the fractions 1 to 7 used in Experimental Examples 1 to 7, the relationship between the reaction completion time of the hydroformylation reaction and the content (GC area%) of the cyclic monoene was illustrated in FIG. 2A.

The relationship between the reaction completion time of the hydroformylation reaction and the content (GC area%) of vinylnorbornene, i.e., the vinyl group-containing cyclic monoene having 9 carbons was illustrated in FIG. 2B.

For the same fractions 1 to 7, the relationship between the TCDDD yield of the hydroformylation reaction and the content (GC area%) of the cyclic monoene was illustrate in FIG. 3A.

Furthermore, for the same fractions 1 to 7, the relationship between the TCDDD yield of the hydroformylation reaction and the content (GC area%) of vinylnorbornene, i.e., the vinyl group-containing cyclic monoene having 9 carbons was illustrated in FIG. 3B.

For the fractions 7 to 14 used in Experimental Examples 7 to 14 and the commercially available DCPD used in Experimental Example 15 for which no vinylnorbornene was detected, i.e., the GC area% was 0.0%, the relationship between the reaction completion time of the hydroformylation reaction and the content (GC area%) of the high-boiling-point byproduct was illustrated in FIG. 4.

Furthermore, for the same fractions 7 to 14 and the commercially available DCPD, the relationship between the TCDDD yield of the hydroformylation reaction and the content (GC area%) of the high-boiling-point byproduct was illustrated in FIG. 5.

The fractions 2 to 14 used in Experimental Examples 2 to 14 were liquid at normal temperature (25°C) and had excellent handleability.

As is clear from FIGS. 2A and 2B, in the case where the content (GC area%) of the cyclic monoene in the raw material compound (fraction) is 14.0 GC area% or less, and preferably 12.0 GC area% or less, and particularly the content (GC area%) of vinylnorbornene, i.e., the vinyl group-containing cyclic monoene having 9 carbons is 3.0 GC area% or less, when the content of the cyclic monoene, especially the content of vinylnorbornene, is smaller, the reaction completion time in the hydroformylation reaction is shorter, and the reaction efficiency is excellent.

As is clear from FIGS. 3A and 3B, in the case where the content (GC area%) of the cyclic monoene in the raw material compound (fraction) is 14.0 GC area% or less, and preferably 12.0 GC area% or less, and particularly the content (GC area%) of vinylnorbornene is 3.0 GC area% or less, and preferably 1.5 GC area% or less, when the content of the cyclic monoene, especially the content of vinylnorbornene, is smaller, the TCDDD yield is higher.

Thus, it is found that the content of the cyclic monoene, especially the vinyl group-containing cyclic monoene having 9 carbons, is preferably equal to or lower than the upper limit value described above.

These results indicate that a threshold is preferably set to the content of the cyclic monoene in the cyclic diene-containing composition, especially the vinyl group-containing cyclic monoene having 9 carbons such as vinylnorbornene, and the content is preferably controlled to be equal to or lower than this threshold.

As is clear from FIG. 4 and FIG. 5, even in the case where the content (GC area%) of the cyclic monoene in the raw material compound (fraction) is 14.0 GC area% or less, and preferably 12.0 GC area% or less, and particularly the content (GC area%) of vinylnorbornene is 3.0 GC area% or less, when the content (GC area%) of the high-boiling-point byproduct is higher, the reaction completion time in the hydroformylation reaction is longer, and the TCDDD yield tends to decrease. Thus, it is found that the content of the high-boiling-point byproduct is preferably equal to or lower than the upper limit value described above.

These results indicate that a threshold is preferably set to the content of the high-boiling-point byproduct in the cyclic diene-containing composition, and the content is preferably controlled to be equal to or lower than this threshold.

Because the content (GC area%) of the cyclic monoene in each of the fraction 1 used in Experimental Example 1 and the fraction 2 used in Experimental Example 2 was higher than that of the other fractions 3 to 13 and, especially, the content of vinylnorbornene in the fraction 1 was higher than 3.0 GC area%, the reaction completion time of the hydroformylation reaction was longer than that in the case of the other fractions 2 to 13, and the reaction efficiency was unsatisfactory.

Because the content (GC area%) of the high-boiling-point byproduct was higher than 25.0 GC area% in the fraction 14 used in Experimental Example 14, the reaction completion time of the hydroformylation reaction was long, and the TCDDD yield was low.

The commercially available DCPD used in Experimental Example 15 had a low content (GC area%) of the high-boiling-point byproduct and a high DCPD purity, and thus the DCPD was solid at normal temperature (25°C) and exhibited poor handleability.

Although the present invention has been described in detail with reference to specific embodiments, it will be apparent to those skilled in the art that various modifications can be made without departing from the spirit and scope of the present invention.

The present application is based on JP 2023-070230 filed on April 21, 2023, JP 2023-077353 filed on May 9, 2023, and JP 2023-173116 filed on October 4, 2023, which are incorporated herein by reference in their entireties.

### Reference Signs List

1 Dimerization tank
2 Distillation column
3 Low-boiling-point component removal column
4 High-boiling-point component removal column

## Claims

1. A cyclic diene-containing composition having a content of a cyclic diene of 99.5 GC area% or less and a content of a cyclic monoene of 14.0 GC area% or less.

2. The cyclic diene-containing composition according to claim 1, wherein a content of a high-boiling-point byproduct having a higher boiling point than the cyclic diene is 0.3 GC area% or more.

3. The cyclic diene-containing composition according to claim 1, wherein the cyclic monoene contains a vinyl group.

4. The cyclic diene-containing composition according to claim 3, wherein the cyclic monoene contains a vinyl group-containing cyclic monoene having 9 carbons.

5. The cyclic diene-containing composition according to claim 4, wherein a content of the vinyl group-containing cyclic monoene having 9 carbons is 3.0 GC area% or less.

6. The cyclic diene-containing composition according to claim 1, wherein the content of the cyclic diene is 60 GC area% or more.

7. The cyclic diene-containing composition according to claim 1, wherein the content of the cyclic monoene is 0.001 GC area% or more.

8. The cyclic diene-containing composition according to claim 1, wherein a content of a high-boiling-point byproduct having a higher boiling point than the cyclic diene is 25 GC area% or less.

9. The cyclic diene-containing composition according to claim 2, wherein the content of the high-boiling-point byproduct is measured by measurement method 1 described below:
Measurement method 1
the method including measurement of a total content of a peak at a retention time from 15.0 minutes to 30.0 minutes by analysis under GC measurement conditions below:
GC measurement conditions
GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C.

10. The cyclic diene-containing composition according to claim 1, wherein the cyclic diene-containing composition has fluidity at 25°C.

11. The cyclic diene-containing composition according to claim 1, wherein the cyclic diene is a polycyclic diene.

12. The cyclic diene-containing composition according to claim 11, wherein the polycyclic diene is dicyclopentadiene.

13. The cyclic diene-containing composition according to claim 1, wherein the cyclic monoene is a polycyclic monoene.

14. The cyclic diene-containing composition according to claim 13, wherein the polycyclic monoene contains 5-vinyl-2-norbornene.

15. The cyclic diene-containing composition according to claim 1, wherein the cyclic diene-containing composition is a composition produced by purification and separation of a hydrocarbon decomposition product obtained by thermal decomposition of a hydrocarbon-containing composition.

16. The cyclic diene-containing composition according to claim 15, wherein the hydrocarbon-containing composition is naphtha.

17. A method for producing an aldehyde, the method comprising subjecting the cyclic diene-containing composition described in any one of claims 1 to 16 to a hydroformylation reaction to produce an aldehyde corresponding to the cyclic diene.

18. A method for producing an alcohol, the method comprising producing an aldehyde by the method described in claim 17, and producing a corresponding alcohol from the aldehyde.

19. A method for producing an aldehyde, the method comprising subjecting a cyclic diene in a cyclic diene-containing composition to a hydroformylation reaction to produce a corresponding aldehyde, wherein
the method comprises controlling a content of a cyclic monoene contained in the cyclic diene-containing composition to a predetermined threshold (1) or less.

20. The method for producing an aldehyde according to claim 19, wherein the predetermined threshold (1) is 14.0 GC area%.

21. The method for producing an aldehyde according to claim 19, wherein the method comprises controlling a content of a high-boiling-point byproduct contained in the cyclic diene-containing composition and having a higher boiling point than the cyclic diene to a predetermined threshold (2) or more.

22. The method for producing an aldehyde according to claim 21, wherein the predetermined threshold (2) is 0.3 GC area%.

23. The method for producing an aldehyde according to claim 19, wherein a content of the cyclic diene contained in the cyclic diene-containing composition is 99.5 GC area% or less.

24. The method for producing an aldehyde according to claim 19, wherein the cyclic monoene contains a vinyl group.

25. The method for producing an aldehyde according to claim 24, wherein the cyclic monoene contains a vinyl group-containing cyclic monoene having 9 carbons.

26. The method for producing an aldehyde according to claim 25, wherein a content of the vinyl group-containing cyclic monoene having 9 carbons in the cyclic diene-containing composition is 3.0 GC area% or less.

27. The method for producing an aldehyde according to claim 19, wherein a content of the cyclic diene contained in the cyclic diene-containing composition is 60 GC area% or more.

28. The method for producing an aldehyde according to claim 19, wherein a content of the cyclic monoene contained in the cyclic diene-containing composition is 0.001 GC area% or more.

29. The method for producing an aldehyde according to claim 19, wherein a content of a high-boiling-point byproduct contained in the cyclic diene-containing composition and having a higher boiling point than the cyclic diene is 25 GC area% or less.

30. The method for producing an aldehyde according to claim 21, wherein a content of the high-boiling-point byproduct is measured by measurement method 1 described below:
Measurement method 1
the method including measurement of a total content of a peak at a retention time from 15.0 minutes to 30.0 minutes by analysis under GC measurement conditions described below:
GC measurement conditions
GC apparatus: gas chromatograph
Detector: flame ionization detector
Carrier gas: helium (column flow rate: 1.65 mL/min)
Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C.

31. The method for producing an aldehyde according to claim 19, wherein the cyclic diene is a polycyclic diene.

32. The method for producing an aldehyde according to claim 31, wherein the polycyclic diene is dicyclopentadiene.

33. The method for producing an aldehyde according to claim 19, wherein the cyclic monoene is a polycyclic monoene.

34. The method for producing an aldehyde according to claim 33, wherein the polycyclic monoene contains 5-vinyl-2-norbornene.

35. The method for producing an aldehyde according to claim 19, wherein the cyclic diene-containing composition is a composition produced by distillation and purification of a hydrocarbon decomposition product obtained by thermal decomposition of a hydrocarbon-containing composition.

36. The method for producing an aldehyde according to claim 35, wherein the hydrocarbon-containing composition is naphtha.

37. The method for producing an aldehyde according to claim 35, wherein the method comprises controlling a condition of the distillation and purification so that the content of the cyclic monoene contained in the cyclic diene-containing composition is adjusted to the predetermined threshold (1) or less.

38. The method for producing an aldehyde according to claim 35, wherein the method comprises controlling a condition of the distillation and purification so that a content of the high-boiling-point byproduct contained in the cyclic diene-containing composition is adjusted to a predetermined threshold (2) or more.

39. The method for producing an aldehyde according to claim 37 or 38, wherein, in the distillation and purification, a first distillation column and a second distillation column are included, the number of theoretical plates of the first distillation column is 10 or more and 20 or less, and the number of theoretical plates of the second distillation column is 10 or more and 20 or less.

40. The method for producing an aldehyde according to claim 39, wherein a reflux ratio of the first distillation column is 20 or more and 30 or less, and a reflux ratio of the second distillation column is 1.0 or more and 1.5 or less.

41. A method for producing an alcohol, the method comprising producing an aldehyde by the method described in any one of claims 19 to 38, and producing a corresponding alcohol from the aldehyde.

42. A method for producing an alcohol, the method comprising producing an aldehyde by the method described in claim 39, and producing a corresponding alcohol from the aldehyde.

43. A method for producing a cyclic diene-containing composition, the method comprising performing distillation and purification of a hydrocarbon decomposition product obtained by thermal decomposition of a hydrocarbon-containing composition to produce a cyclic diene-containing composition containing a cyclic diene, wherein
the method comprises controlling a content of a cyclic monoene contained in the cyclic diene-containing composition to a predetermined threshold (1) or less.

44. The method for producing a cyclic diene-containing composition according to claim 43, wherein the method comprises controlling a content of a high-boiling-point byproduct contained in the cyclic diene-containing composition and having a higher boiling point than the cyclic diene to a predetermined threshold (2) or more.

45. The method for producing a cyclic diene-containing composition according to claim 43, wherein the predetermined threshold (1) is 14.0 GC area%.

46. The method for producing a cyclic diene-containing composition according to claim 44, wherein the predetermined threshold (2) is 0.3 GC area%.

47. The method for producing a cyclic diene-containing composition according to claim 43, wherein the method comprises controlling a condition of the distillation and purification so that the content of the cyclic monoene contained in the cyclic diene-containing composition is adjusted to the predetermined threshold (1) or less.

48. The method for producing a cyclic diene-containing composition according to claim 44, wherein the method comprises controlling a condition of the distillation and purification so that the content of the high-boiling-point byproduct contained in the cyclic diene-containing composition is adjusted to the predetermined threshold (2) or more.

49. The method for producing a cyclic diene-containing composition according to claim 47 or 48, wherein, in the distillation and purification, a first distillation column and a second distillation column are included, the number of theoretical plates of the first distillation column is 10 or more and 20 or less, and the number of theoretical plates of the second distillation column is 10 or more and 20 or less.

50. The method for producing a cyclic diene-containing composition according to claim 49, wherein a reflux ratio of the first distillation column is 20 or more and 30 or less, and a reflux ratio of the second distillation column is 1.0 or more and 1.5 or less.

51. The method for producing a cyclic diene-containing composition according to claim 43, wherein a content of the cyclic diene contained in the cyclic diene-containing composition is 99.5 GC area% or less.

52. The method for producing a cyclic diene-containing composition according to claim 43, wherein the cyclic monoene contains a vinyl group.

53. The method for producing a cyclic diene-containing composition according to claim 52, wherein the cyclic monoene contains a vinyl group-containing cyclic monoene having 9 carbons.

54. The method for producing a cyclic diene-containing composition according to claim 53, wherein a content of the vinyl group-containing cyclic monoene having 9 carbons in the cyclic diene-containing composition is 3.0 GC area% or less.

55. The method for producing a cyclic diene-containing composition according to claim 43, wherein a content of the cyclic diene contained in the cyclic diene-containing composition is 60.0 GC area% or more.

56. The method for producing a cyclic diene-containing composition according to claim 43, wherein a content of the cyclic monoene contained in the cyclic diene-containing composition is 0.001 GC area% or more.

57. The method for producing a cyclic diene-containing composition according to claim 43, wherein a content of a high-boiling-point byproduct contained in the cyclic diene-containing composition and having a higher boiling point than the cyclic diene is 25 GC area% or less.

58. The method for producing a cyclic diene-containing composition according to claim 44, wherein a content of the high-boiling-point byproduct is measured by measurement method 1 described below:
Measurement method 1
the method including measurement of a total content of a peak at a retention time from 15.0 minutes to 30.0 minutes by analysis under GC measurement conditions described below:
GC measurement conditions
GC apparatus: gas chromatograph
Detector: flame ionization detector
Carrier gas: helium (column flow rate: 1.65 mL/min) Column: capillary column (size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C.

59. The method for producing a cyclic diene-containing composition according to claim 43, wherein the cyclic diene is a polycyclic diene.

60. The method for producing a cyclic diene-containing composition according to claim 59, wherein the polycyclic diene is dicyclopentadiene.

61. The method for producing a cyclic diene-containing composition according to claim 43, wherein the cyclic monoene is a polycyclic monoene.

62. The method for producing a cyclic diene-containing composition according to claim 61, wherein the polycyclic monoene contains 5-vinyl-2-norbornene.

63. The method for producing a cyclic diene-containing composition according to claim 43, wherein the hydrocarbon-containing composition is naphtha.

64. A method for producing an aldehyde, the method comprising producing a cyclic diene-containing composition by the method described in any one of claims 43 to 48 and claims 51 to 63, and producing a corresponding aldehyde from a cyclic diene contained in the cyclic diene-containing composition.

65. A method for producing an alcohol, the method comprising producing an aldehyde by the method described in claim 64, and producing a corresponding alcohol from the aldehyde.

66. A method for producing an aldehyde, the method comprising producing a cyclic diene-containing composition by the method described in claim 49, and producing a corresponding aldehyde from a cyclic diene contained in the cyclic diene-containing composition.

67. A method for producing an alcohol, the method comprising producing an aldehyde by the method described in claim 66, and producing a corresponding alcohol from the aldehyde.
